# EUROPÄISCHE PATENTSCHRIFT

(11) **EP 1 274 462 B1**
(45) Veröffentlichungstag und Bekanntmachung des Hinweises auf die Patenterteilung: **19.11.2008**
(21) Anmeldenummer: 01936172.4
(22) Anmeldetag: 03.04.2001
(51) Int. Cl.: A61K 47/48, C12N 15/87

(54) **GEZIELTE TRANSFEKTION VON ZELLEN MITTELS BIOTINYLIERTEM DENDRIMER**
TARGETED TRANSFECTION OF CELLS USING A BIOTINYLATED DENDRIMER
TRANSFECTION CIBLEE DE CELLULES PAR DENDRIMERE BIOTINYLE

(30) Priorität: 05.04.2000 DE 10016881
(43) Veröffentlichungstag der Anmeldung: 15.01.2003
(73) Patentinhaber: QIAGEN GmbH, 40724 Hilden (DE)
(72) Erfinder: WEBER, Martin, 42799 Leichlingen (DE); DENNIG, Jörg, 40764 Langenfeld (DE); ERBACHER, Christoph, 42781 Haan (DE)
(74) Vertreter: Vossius & Partner
(86) Internationale Anmeldenummer: PCT/EP2001/003746
(87) Internationale Veröffentlichungsnummer: WO 2001/076633

(56) Entgegenhaltungen:
- WO-A-93/19768
- WO-A-95/02397
- WO-A-96/00295
- US-A- 5 714 166
- TANG M X ET AL: "IN VITRO GENE DELIVERY BY DEGRADED POLYAMIDOAMINE DENDRIMERS" BIOCONJUGATE CHEMISTRY, AMERICAN CHEMICAL SOCIETY, WASHINGTON, US, Bd. 7, Nr. 6, 1. November 1996 (1996-11-01), Seiten 703-714, XP000638685 ISSN: 1043-1802 in der Anmeldung erwähnt
- WILBUR ET AL.: BIOCONJUGATE CHEMISTRY, Bd. 9, Nr. 6, 1998, Seiten 813-825, XP000786597
- SCHOEMAN ET AL.: J. DRUG TARGETING, Bd. 2, 1995, Seiten 509-516, XP001032675
- HAENSLER & SZOKA: BIOCONJ. CHEM., Bd. 4, 1993, Seiten 372-379, XP000395057

## Beschreibung

Die vorliegende Erfindung betrifft Verfahren zur gezielten Transfektion von Zellen sowie Zusammensetzungen, die für solche Verfahren verwendet werden können.

Aus dem Stand der Technik sind verschiedene Verfahren bekannt, genetisches Material in Zellen einzubringen. Zur Transfektion in vivo werden im wesentlichen zwei Methoden angewendet, Virus-vermittelter Gentransfer und die Transfektion mittels kationischer Liposomen. Als virale Vektoren werden Adenoviren und Retroviren benutzt, die in der Lage sind, nichtvirale DNA in mitotisch aktive Zellen zu transfizieren. Die kationischen Liposomen interagieren mit dem negativ geladenen Phosphat-Rückgrat der DNA. Der resultierende Komplex wird zu den Zellen gegeben und von den Zellen endocytiert. Zur Transfektion in vitro stehen zusätzliche Methoden zur Verfügung, zum Beispiel Calcium-Phosphat-Präzipitation, DEAE(Diethylaminoethyl)-Dextran-Transfektion, und Elektroporation. Bei der Calcium-Phosphat-Methode wird DNA mit CaCl₂ in einem Phosphat-Puffer gemischt. Der entstehende Calcium-Phosphat-Komplex bindet an die Zellmembran und wird endocytiert. Das positiv geladene DEAE-Dextran interagiert mit dem negativ geladenen Phosphat-Rückgrat der DNA. Der DEAE-Dextran-DNA-Komplex bindet an die Zelloberfläche und wird endocytiert. Bei der Elektroporation werden Zellen mit DNA gemischt. Für einen kurzen Moment wird eine hohe Spannung angelegt. In der Zellmembran entstehen Poren, durch die die DNA aufgenommen wird.

Die genannten Verfahren sind mit verschiedenen Nachteilen verbunden. So ermöglichen virale Vektoren zwar die effiziente Transfektion von Zellen auch *in vivo.* Ihr biologisches Gefahrenpotential ist aber sehr hoch. Die Effizienz beim Einsatz kationischer Liposomen *in vivo* ist sehr niedrig. Außerdem ermöglichen beide invivo-Verfahren keine gezielte Transfektion von einzelnen Zelltypen im Gewebeverband. Bei den in-vitro-Anwendungen besteht insbesondere das Problem, daß Suspensionszellinien (insbesondere T- und B-Zellinien) mit den chemischen Transfektionsreagenzien und -Methoden nur sehr schwer transfiziert werden können. Im Gegensatz zu adhärenten Zellen, bei denen oft Effizienzen von über 50 % transfizierten Zellen erreicht werden können, liegen die Effizienzen bei Suspensionszellen im allgemeinen bei 0 % bis 5 % transfizierten Zellen. Bei der Elektroporation sind die Transfektionseffizienzen in Suspensionszellen im allgemeinen ähnlich niedrig. Die Methode bietet aber oft die Möglichkeit, solche Zellinien zu transfizieren, die sich durch andere Methoden überhaupt nicht transfizieren lassen. Bei dieser Methode sterben jedoch durch die Transfektion 50 % bis 70 % der Zellen. Ebenso ist die Menge an DNA und Zellen pro Ansatz, die für diese Transfektionsmethode benötigt werden, deutlich höher als für andere Methoden. Retrovirale Vektoren ermöglichen zwar die effiziente Transfektion von Suspensionszellen. Die Methode ist aber sehr kompliziert und aufwendig. Vor allem besitzen retrovirale Vektoren ein hohes biologisches Gefahrenpotential.

Dendrimere sind dreidimensionale Polymere, die aus reiterativen Sequenzen um einen Kern herum aufgebaut und in verschiedenen Molekulargewichten und Größen herstellbar sind (Tomalia et al., Angew Chem Int Ed 1990,29:138). Aus der WO 95/02397 ist die Komplexbildung von Dendrimeren mit Nukleinsäuren und die Verwendung von Dendrimeren zur Transfektion von Zellen bekannt. Tang et al., Bioconjugate Chem 1996, 7:703-714 beschreiben die Aktivierung von Polyamidoamin-Dendrimerendurch thermische Degradation zur Verbesserung der Transfektionseffizienz. Die Verwendung von Dendrimeren allein als Transfektionsreagenzien ist aber ebenfalls mit den oben beschriebenen Nachteilen behaftet.

Schoeman et al., J Drug Target 1995, 2(6):509-516 beschreiben ein System zum gezielten Transfer von DNA in Zellen, bei dem biotinyliertes Polylysin verwendet wird, das über Streptavidin an biotinyliertes Transferrin gebunden wird.

Haensler und Szoka, Bioconjug Chem 1993, 4(5): 372-379 beschreiben Transfektionsbedingungen, wie Dendrimer/DNA-Verhältnisse oder Dendrimerdiameter, für die Verwendung von Starburst Dendrimeren.

Wilbur et al., Bioconjug Chem 1998, 9(6):813-825 beschreiben Biodistributionsexperimente mit biotinylierten Starburst-Dendrimeren.

Aufgabe der vorliegenden Erfindung war die Bereitstellung verbesserter Transfektionsverfahren und der dafür erforderlichen Mittel, die es insbesondere gestatten, Nukleinsäure auch in schwer transfizierbare Zellen wie Suspensionszellen einzuführen.

Gelöst wird diese Aufgabe durch ein Verfahren zum Einbringen von Nukleinsäure in Zellen mit den Schritten
(a) Mischen einer Nukleinsäure mit einem Dendrimer, wobei ein Anteil der Dendrimermoleküle biotinyliert ist; und der Anteil der Dendrimermoleküle, die biotinyliert sind, an der Gesamtmenge der Dendrimer moleküle 5 bis 20% (Mol/Mol) beträgt.
(b) Mischen des entstandenen Komplexes aus Nukleinsäure und Dendrimer mit einem zweiten Komplex, bestehend aus Avidin oder Streptavidin und einem biotinylierten zielspezifischen Bindungsmolekül;
(c) Inkubation des in Schritt (b) entstandenen Komplexes mit Zellen.

Die Nukleinsäure, für die synonym auch der Begriff Polynukleotid verwendet werden kann, kann eine Ribonukleinsäure (RNA), Deoxyribonukleinsäure (DNA), oder auch gemischte Form sein. Im allgemeinen Fall kann es sich um jeden Typ von Polynukleotid handeln, der ein N-Glykosid oder C-Glykosid einer Purin- oder Pyrimidinbase oder einer modifizierten Purin- oder Pyrimidinbase darstellt, also auch solche Basen enthalten kann, die in der Natur nicht vorkommen. Außer der Base selbst kann auch der Zuckerrest modifiziert sein. Eingeschlossen sind Nukleinsäuren mit modifizierter Verknüpfung der Untereinheiten, etwa Phosphorothioat- oder -amidat-Verknüpfungen. Die Nukleinsäure kann einzel-, doppel- oder mehrsträngig, linear oder zirkulär sein. Sie kann einem in einer Zelle vorkommenden Molekül entsprechen wie etwa genomische DNA oder Boten-RNA (mRNA), oder in vitro erzeugt werden wie Komplementär-DNA (cDNA), Gegenstrang-RNA (aRNA), oder synthetische Nukleinsäuren. Die Nukleinsäure kann aus wenigen Untereinheiten, mindestens zwei Untereinheiten, bevorzugt acht oder mehr Untereinheiten bestehen wie etwa Oligonukleotide, mehreren hundert Untereinheiten bis hin zu mehreren tausend Untereinheiten wie etwa bestimmte Expressionsvektoren. Bevorzugt enthält die Nukleinsäure die kodierende Information für ein Polypeptid in funktioneller Zusammenhang mit regulatorischen Sequenzen, die die Expression des Polypeptids in der Zelle erlauben, in die die Nukleinsäure eingebracht wird. So ist die Nukleinsäure in einer bevorzugten Ausführungsform ein Expressions-vektor. In einer anderen Ausführungsform ist sie ein Gegenstrang (antisense)-Oligonukleotid, das beispielsweise durch Hybridisierung mit einer anderen Nukleinsäure in der Zelle, in die dieses Gegenstrang-Oligonukleotid eingebracht wird, die Expression eines bestimmten Gens unterdrücken kann. Der erfindungsgemäße Komplex kann auch Gemische verschiedener Nukleinsäuren enthalten.

Dendrimer im Sinne der vorliegenden Erfindung ist ein verzweigtes Polymer, das eine dreidimensionale, hochgeordnete Verbindung darstellt, wobei verzweigte oligomere/polymere Sequenzen um ein Kernmolekül herum durch reiterative Reaktionsequenzen aufgebaut werden können, und das durch geeignete funktionale Endgruppen unter bestimmten Bedingungen eine positiv geladene äußere Oberfläche aufweist (polykationisches Dendrimer). Solche Dendrimere und ihre Herstellung werden beschrieben in WO 84/02705, US 4,507,466, US 4,558,120, US 4,568,737, 4,587,329, US 4,631,337, US 4,694,064, US 4,713,975, US 4,737,550, US 4,871,779, 4,857,599, EP 0 234 408, EP 0 247 629, EP 0 271 180, und insbesondere Tang et al., supra, WO 95/02397, und Tomalia et al., supra.

Geeignete Kernmoleküle weisen mindestens zwei reaktive Gruppen auf, über die die oligomeren/polymeren Sequenzen mit dem Kern verknüpft werden können, oder sie sind zur Einführung solcher reaktiven Gruppen geeignet. Beispiele für solche reaktiven Gruppen sind Hydroxyl-, Ether-, Amino-, Imino-, Amido-, Imido-, Ammonium-, Halogen-, Carboxyl-, Carboxyhalid-, Sulfhydrylgruppen etc. Bevorzugte Kernmoleküle sind Ammoniak, Tris-(2-aminoethyl)amin (TAEA), Lysin, Ornithin, Pentaerythritol, und Ethylendiamin (EDA).

Um diese Kernmoleküle können die verzweigten oligomeren/polymeren Sequenzen durch stufenweise Addition von Monomeren aufgebaut werden. Jeder Reaktionszyklus, der neue Verzweigungspunkte erzeugt, generiert dadurch eine höhere "Generation" des Dendrimers. Bevorzugt sind dabei pharmazeutisch gut verträgliche Sequenzen wie zum Beispiel Polyamidoamine, herstellbar durch die Reaktion eines Alkylesters einer α,β-ethylenisch ungesättigten Carbonsäure oder eines α,β-ethylenisch ungesättigten Amides mit einem Alkylen-Polyamin oder einem Polyalkylen-Polyamin.

Die terminalen Gruppen der oligomeren/polymeren Sequenzen, die die äußere Oberfläche des Dendrimers bilden, sollen die Eigenschaft haben, daß sie unter geeigneten Bedingungen positive Ladungen aquirieren können, zum Beispiel in wäßriger Lösung bei physiologischen pH-Werten. Beispiele für solche Gruppen sind primäre, sekundäre, tertiäre und quaternäre aliphatische oder aromatische Amine, Guanidiniumgruppen, oder Azole, die auch mit S oder O substituiert sein können, oder Kombinationen davon. Die terminalen kationischen Gruppen können zu einem Anteil von 10 bis 100% aller terminalen Gruppen vorhanden sein, bevorzugt 50 bis 100%. Das Dendrimer kann auch nicht-kationische terminale Gruppen in einem Anteil von 0 bis 90% aufweisen, z.B. Hydroxyl-, Cyano-, Carboxyl-, Sulfhydryl-, Amid- oder Thioethergruppen.

Für die vorliegende Erfindung gut geeignete Dendrimere sind beispielsweise Polyamidoamin(PAMAM)-Dendrimere, die um Ammoniak, Tris-(2-aminoethyl)amin (TAEA) oder Ethylendiamin (EDA) als Kernmoleküle durch stufenweise Addition der beiden Monomere Methacrylat und Ethylendiamin aufgebaut werden können (Tang et al., supra). Die terminalen Gruppen eines solchen Dendrimers sind bevorzugt primäre Aminogruppen. Bevorzugt sind dabei PAMAM-Dendrimere der 5., 6. oder 7. Generation, insbesondere der 6. Generation gemäß Tang et al., supra. Die theoretischen Molekulargewichte, Zahl der terminalen Amine und hydrodynamischen Radien solcher PAMAM-Dendrimere können der Publikation Tang et al., supra, entnommen werden.

Die Dendrimere sind bevorzugt nicht völlig intakte oder hochsymmetrische Moleküle gemäß ihrer Idealstruktur, sondern weisen in einem gewissen Ausmaß strukturelle Defekte, Fehlstellen oder Abweichungen von der Idealstruktur auf. Es hat sich gezeigt, daß solche strukturellen Defekte die Brauchbarkeit von Dendrimeren als Transfektionsreagenzien signifikant erhöhen können (Tang et al., supra). Solche Defekte können beispielsweise durch partielle solvolytische Degradation des Dendrimers erzeugt werden. Geeignete Lösungsmittel dafür sind beipielsweise Wasser, 1-Butanol, 2-Butanol, oder 2-Ethoxyethanol. Die Solvolyse kann durch Hitze beschleunigt werden. So führt beispielsweise Kochen in Wasser für 5 bis 15 Stunden, bevorzugt 7 bis12 Stunden, bei einem TAEA-PAMAM-Dendrimer der 6. Generation (6-TAEA-PAMAM) zu einer deutlichen Erhöhung der Transfektionseffizienz. Für ein 5-TAEA-PAMAM liegt der bevorzugte Bereich bei 5 bis 12 Stunden, für ein 6-EDA-PAMAM bei 10 bis 15 Stunden. Die optimalen Refluxzeiten in anderen Lösungsmitteln können entsprechend variieren, z.B. in wasserfreiem 1-Butanol 20 bis 60 Stunden für 6-TAEA-PAMAM, bevorzugt 30 bis 50 Stunden, besonders bevorzugt 40-50 Stunden. Alternativ zur solvolytischen Degradation können die genannten strukturellen Defekte auch bereits bei der Synthese erzeugt werden, beispielsweise durch anteilige Zugabe von Monomeren bei jedem Zyklus, die keine Verzweigungspunkte generieren können oder keine Kettenverlängerung zulassen, z.B. *N*,*N*'-Dimethylacrylamid. Dendrimere mit strukturellen Defekten, Fehlstellen oder Abweichungen von der Idealstruktur in dem genannten Sinne, insbesondere solche, die durch partielle Solvolyse erzeugt werden können, sollen hier als aktivierte Dendrimere bezeichnet werden. Es wird angenommen, daß die bessere Brauchbarkeit von aktivierten Dendrimeren als Transfektionsreagenzien mit ihrer größeren sterischen Flexibilität zusammenhängt.

Ein aktiviertes Dendrimer, das sich sehr gut für die vorliegende Erfindung eignet, ist in dem Produkt SuperFect^{®} der Firma Qiagen GmbH, Hilden, Deutschland, enthalten.

Der Anteil der biotinylierten Dendrimermoleküle an der Gesamtmenge der Dendrimermoleküle im Komplex ist 0.5 bis 50 % (Mol/Mol), bevorzugt 5 bis 20 % (Mol/Mol), ganz besonders bevorzugt 10 bis15 % (Mol/Mol). Vorteilhaft kann ein vorgefertigtes Gemisch aus biotinyliertem und nicht-biotinyliertem Dendrimer zur Bildung des erfindungsgemäßen Komplexes mit der Nukleinsäure umgesetzt werden, doch können die drei Komponenten auch in anderer Reihenfolge oder gleichzeitig vermischt werden. Ein solches Gemisch aus biotinyliertem und nicht-blotinyliertem Dendrimer kann hergestellt werden, indem biotinyliertes und nicht-biotinyliertes Dendrimer beispielsweise in Massenverhäftnissen von 1:1 bis 1:256, bevorzugt 1: 2 bis 1:128, besonders bevorzugt 1:4 bis 1:24, ganz besonders bevorzugt 1:6 bis 1:10 vermischt werden. Die Vermischung kann zweckmäßig durch Vermischen wäßriger Lösungen der Komponenten erfolgen.

Unter biotinyliertem Dendrimer soll hierbei eine Dendrimerzusammensetzung verstanden werden, in der mindestens 80%, bevorzugt mindestens 90 %, besonders bevorzugt mindestens 95%, ganz besonders bevorzugt mindestens 98% oder 99% der Dendrimermoleküle mit einem oder mehreren Biotinmolekülen verknüpft sind.

Biotin (Merck Index No. 1272, 12^{th} Ed., Merck & Co., Whitehouse Station, NJ, USA) und Methoden zur Biotinylierung, d.h. zur Verknüpfung von Biotin oder Biotinderivaten mit anderen Substanzen, meist unter Verwendung eines aktivierten Biotins oder Biotinderivats, sind aus dem Stand der Technik bekannt (Bayer et al., Anal Biochemistry 1985, 149: 529-536; Bengtstrom et al., Nucleosides and Nucleotides 1990, 9: 123; Pantano and Kuhr, Anal Chem 1993, 65: 623-630; Bayer and Wilchek, Method of Biochemical Analysis 1980, 26: 1-45; Hofmann et al., Avidin binding of carboxyl-substituted biotin and analogues. Biochemistry 1982, 21: 978-984; Leary et al., Proc Natl Acad Sci USA 1983, 80: 4045-4049; Costello et al., Clin Chem 1979, 25: 1572-1580; Spiegel and Wilchek, J Immunol 1981, 127: 572-575; Rosenberg et al., J Neurochem 1986, 46: 641-648).

Unter aktiviertem Biotin oder aktiviertem Biotinderivat sollen hier Verbindungen verstanden werden, die sich vom Biotin ableiten, und die durch Einführung einer reaktiven Gruppe, insbesondere einer Abgangsgruppe an einer Carboxyfunktion der Seitenkette des Biotins für Biotinylierungsreaktionen geeignet sind. In den Begriff des Biotinderivats sind insbesondere eingeschlossen modifizierte Formen des Biotins, in denen beispielsweise die Seitenkette des Biotins verlängert wurde, sodaß daraus eine Abstandshalterfunktion (Spacer) resultiert. Dabei können auch andere funktionelle Gruppen eingeführt werden, die eine kovalente Verknüpfung des Biotins mit Dendrimeren, Proteinen, oder anderen Substanzen erlauben. Ein solches Derivat ist beispielsweise 6-(Biotinamido)hexansäure (Biotinamidocapronsäure) und deren aktivierte Derivate, wie z.B. Biotinamidocapronsäure-*N*-hydroxysuccinimidester.

Der Grad der Biotinylierung der biotinylierten Dendrimermoleküle, d.h. die Zahl der Biotinmoleküle, die mit einem Dendrimermolelkül verknüpft ist, beträgt vorzugsweise 1 oder mehr. Bevorzugt ist biotinyliertes Dendrimer, das durch Umsetzung von Dendrimer mit einem ein- bis 64fachen molaren Überschuß an Biotin hergestellt wurde, besonders bevorzugt mit einem 2- bis 16fachen, besser noch 2- bis 12fachen Überschuß, beispielsweise einem 3- bis 5fachen, insbesondere einem 4fachen Überschuß an Biotin. Für die Biotinylierung von Dendrimeren können an sich bekannte Standardverfahren angewendet werden, zum Beispiel die Umsetzung von Dendrimer mit N-(+)-Biotinyl-6-Aminocapronsäure-N-Succinimidylester, einem aktivierten Biotinderivat, das zusätzlich ein Hexansäuregerüst als Abstandshalter (Spacer) enthält (Hermanson G T, Bioconjugate Techniques, Academic Press, London, Großbritannien, 1996, Seiten 575-580).

Geeignete Mischungsverhältnisse für Nukleinsäure zu Dendrimer sind von 10:1 (Gew/Gew) bis 1:50 (Gew/Gew), bevorzugt 1:2 bis 1:20, für Suspensionszellen beispielsweise bevorzugt von 1:2 bis 1:10 (Gew/Gew), ganz besonders bevorzugt etwa 1:3. Der Fachmann kann durch Routineexperimente für das jeweilige Transfektionsproblem optimierte Mischungsverhältnisse ermitteln.

Die Mischung von Nukleinsäure und Dendrimer erfolgt unter Bedingungen, unter denen es zu der Ausbildung eines Komplexes zwischen Nukleinsäure und Dendrimer kommt. Dem Fachmann sind solche Bedingungen bekannt. Zweckmäßig kann diese Mischung durch Vereinigung wäßriger Lösungen der Komponenten ausgeführt werden, wobei eine oder beide dieser Lösungen auch gepuffert sein und ggf. weitere Zusätze enthalten können.

Durch Vermischen der Nukleinsäure und des partiell biotinylierten Dendrimers (d.h. beispielsweise einem Gemisch von biotinyliertem Dendrimer und nicht-biotinyliertem Dendrimer in den oben angegebenen Mengenverhältnissen) unter geeigneten Bedingungen entsteht ein Komplex, in dem die Komponenten durch nicht-kovalente Wechselwirkungen miteinander assoziiert sind.

In einem solchen Komplex können gegebenenfalls Dendrimer und Nukleinsäure auch durch kovalente Bindungen miteinander verknüpft werden.

Erfindungsgemäß kann der so entstandene Komplex aus Nukleinsäure und partiell biotinyliertem Dendrimer unter geeigneten Bedingungen mit einem zweiten Komplex, bestehend aus Avidin oder Streptavidin und einem biotinylierten Zielspezifischen Bindungsmolekül gemischt und auf diese Weise ein größerer Komplex hergestellt werden, in dem das Nukleinsäure-Dendrimer-Addukt über das Avidin-Biotin-System mit einem zielspezifischen Bindungsmolekül verknüpft wird. Ein solcher Komplex erlaubt seine zielgerichtete Bindung an die Oberfläche von Zellen, die einen Bindungspartner oder Rezeptor des Bindungsmoleküls tragen, und damit das zielspezifische Einbringen der gewünschten Nukleinsäure in solche Zellen. Diese Mischung der beiden Komplexe kann zweckmäßig erfolgen, indem geeignete wäßrige Lösungen der beiden Komplexe vereinigt werden, wobei eine oder beide dieser Lösungen gepuffert sein und gegebenenfalls weitere Zusätze enthalten kann.

Avidin ist ein aus dem Stand der Technik bekanntes Glycoprotein, das mit hoher Affinität biotinylierte Substanzen binden kann (Merck Index No. 920, 12^{th} Ed., Merck & Co., Whitehouse Station, NJ, USA); Fuccillo, Application of the avidin-biotin technique in microbiology, BioTechniques 1985, 3: 494; Methods Enzymol Eds. M. Wilchek, E.A. Bayer 1990, 184: 1-671). Es kann beispielsweise aus Hühnereiweiß isoliert werden, hat dann ein Molekulargewicht von etwa 66000 und ist kommerziell erhältlich. Alternativ kann auch Streptavidin, ein Protein mit einem Molekulargewicht von etwa 60000 aus Streptomyces avidinii verwendet werden (Fuccillo, Application of the avidin-biotin technique in microbiology, BioTechniques 1985, 3: 494; Haeuptle et al., Protein isolated from the bacterium Streptomyces avidin, having a high affinity for biotin, J Biol Chem 1983, 258: 305; Avidin-Biotin technology: Methods Enzymol Eds., M. Wilchek, E.A. Bayer 1990, 184: 1-671).

Zielspezifische Bindungsmoleküle, die spezifisch an bestimmte Bindungspartner binden, sind im Stand der Technik in großer Zahl bekannt. Im Sinne der vorliegenden Erfindung sind solche Bindungsmoleküle bevorzugt, die an Bindungspartner oder Rezeptoren binden, die auf der Oberfläche von Zellen exponiert sind, insbesondere solchen Bindungspartnem oder Rezeptoren, die spezifisch von bestimmten Zelltypen exprimiert werden. Weiterhin sind solche zielspezifischen Bindungsmoleküle bevorzugt, die nach Bindung an ihren Bindungspartner oder Rezeptor auf der Zelloberfläche in das Zellinnere intemalisiert werden, beispielsweise durch Rezeptor-vermittelte Endozytose.

Ein solches zielspezifisches Bindungsmolekül ist zum Beispiel das Protein Transferrin, dessen spezifischer Bindungspartner, der Transferrin-Rezeptor, von einer Reihe von Zelltypen, beispielsweise der B-Zelllinie K562 (ATCC CCL-243) exprimiert wird. Transferrin ist ein Eisen transportierendes Protein, das nach der Bindung an seinen Rezeptor aktiv endozytiert wird. Wird Transferrin über das Avidin-Biotin-System an einen Nukleinsäure-Dendrimer-Komplex konjugiert, können mit einem solchen Komplex Zellen gezielt transfiziert werden, die den Transferrinrezeptor exprimieren. Ein anderes Beispiel für ein solches zielspezifisches Bindungsmolekül ist ein Antikörper, der spezifisch für das T-Zell-spezifische Oberflächenantigen CD3 ist. Bindet ein solcher Antikörper an CD3, wird der entstandene Komplex aktiv in die Zelle internalisiert. Bei der Verwendung eines CD3-spezifischen Antikörpers als zielspezifisches Bindungsmolekül für die vorliegende Erfindung lassen sich selektiv T-Zellen transfizieren. Eine T-Zellinie, die CD3 exprimert, ist die Zellinie Jurkat (DSMZ ACC 282). Analog können für die vorliegende Erfindung auch Antikörper als zielspezifische Bindungsmoleküle verwendet werden, die für andere Bindungspartner oder Rezeptoren spezifisch sind, insbesondere für Zelltyp-spezifische Zelloberflächenmarker, die nach Bindung eines Liganden intemalisiert werden. Als Antikörper können außer Immunglobulinen, die Tetramere oder Multimere aus kompletten schweren und leichten Ketten darstellen, auch abgeleitete Antikörpermoleküle verwendet werden, die im Stand der Technik bekannt sind, z.B. Fragmente von Immunglobulinen, die die Antigenbindungsdomäne enthalten (z.B. F_{ab}-Fragmente) oder rekombinante Proteine, die Antigenbindungsdomänen (insbesondere die sogenannten variablen Regionen) von Immunglobulinen ggf. in modifizierter Form enthalten, z.B. humanisierte Antikörper oder einkettige Antikörpermoleküle (scFv). Der Begriff "Antikörper" soll hier alle solchen Moleküle einschließen, die spezifisch an einen Bindungspartner binden und wenigstens eine Aminosäuresequenz enthalten, die homolog zu der variablen Region einer Immunglobulinkette ist.

Ein weiteres Beispiel für ein zielspezifisches Bindungsmolekül ist ein Antikörper gegen CD62E (ELAM-1, E-Selectin), z.B. der monoklonale Antikörper, der von der Hybridoma-Zelllinie H18/7 (ATCC HB-11684) produziert wird. CD62E ist ein Oberflächenmarker für aktivierte vaskuläre Epithelzellen, die eine zentrale Rolle bei Entzündungsprozessen spielen, und ein klinisches Target für die Gentherapie auf diesem Indikationsgebiet (Harari et al., Gene Ther 1999, 6(5):801-7).

Das zielspezifische Bindungsmolekül kann nach an sich bekannten Methoden biotinyliert werden, zum Beispiel mit Biotinamidocapronsäure-*N-*hydroxysuccinimidester in wäßriger Hydrogencarbonatlösung (pH 8.5) (Hofmann, K., et al., Avidin binding of carboxyl-substituted biotin and analogues. Biochemistry, 21, 978-984 (1982)). Bei der Verwendung dieses Biotinderivats wird durch das Capronsäuregerüst gleichzeitig ein Abstandshalter (Spacer) zwischen das Bindungsmolekül und das Biotin eingeführt, um eine optimale Wechselwirkung zwischen Biotin und Avidin oder Streptavidin zu gewährleisten. Ist das zielspezifische Bindungsmolekül ein Protein, zum Beispiel Transferrin oder ein Antikörper, hat sich ein Biotinylierungsgrad von 1 bis 15 Biotinmolekülen pro Bindingsproteinmolekül als zweckmäßig erwiesen, bevorzugt 5 bis 13 Biotinmoleküle, besonders bevorzugt 8 bis 12 Biotinmoleküle pro Proteinmolekül. Der Biotinylierungsgrad kann durch die Wahl eines geeigneten molaren Mengenverhältnisses der Reaktanden der Biotinylierungsreaktion gesteuert werden.

Der Komplex aus biotinyliertem zielspezifischem Bindungsprotein und Avidin oder Streptavidin kann durch Mischung der beiden Komponenten unter geeigneten Bedingungen erfolgen. Solche Bedingungen sind dem Fachmann bekannt. Zweckmäßig kann die Mischung durch Vereinigung wäßriger Lösungen der Komponenten erfolgen, wobei eine oder beide dieser Lösungen gepuffert sein und gegebenenfalls weitere Zusätze enthalten kann.

Als Mengenverhältnis von Nukleinsäure/Dendrimer-Komplex zu Avidin oder Streptavidin hat sich 1:1 bis 100:1 (Gew/Gew) als zweckmäßig erwiesen, ganz besonders bevorzugt jedoch 1.5:1 bis 4:1 (Gew/Gew), insbesondere 2:1 bis 4:1. Für das Mengenverhältnis Nukleinsäure/Dendrimer-Komplex zu zielspezifischem Bindungsmolekül hat sich 1:1 bis 40:1 (Gew/Gew) als zweckmäßig erwiesen, besonders vorteilhaft 2:1 bis 10:1 (Gew/Gew). Sehr gute Ergebnisse konnten beispielsweise mit Mengenverhältnissen von 1:3:2:1 (Nukleinsäure: Dendrimer: Avidin: Bindungsprotein; Gew/Gew/Gew/Gew) erzielt werden.

Für das erfindungsgemäße Verfahren ist die Einhaltung einer bestimmten Reihenfolge der Verfahrensschritte wichtig. Es hat sich gezeigt, daß die vorteilhaften Wirkungen des Verfahrens insbesondere dann zum Tragen kommen, wenn soWohl der Komplex aus Nukleinsäure und Dendrimer als auch der Komplex aus Avidin oder Streptavidin und zielspezifischem Bindungsmolekül vorgefertigt werden, bevor beide Komplexe dann zusammengegeben werden.

Der dabei erhaltene Komplex, der die Komponenten Nukleinsäure, Dendrimer, und zielspezifisches Bindungsmolekül in operativer Weise vereinigt, kann zum Einbringen der Nukleinsäure in Zellen verwendet werden. Zu diesem Zweck wird dieser Komplex, im folgenden auch Transfektionskomplex genannt, mit Zellen inkubiert, d.h. unter geeigneten Bedingungen in Kontakt mit Zellen gebracht.

So können Zellen oder Gewebe in vitro mit dem Transfektionskomplex in einem geeigneten Milieu inkubiert werden. Sollen Kulturzellen transfiziert werden, kann der Transfektionskomplex zum Kulturmedium gegeben werden. Zur Transfektion in vivo kann der Transfektionskomplex in einen tierischen oder pflanzlichen Organismus einschließlich des Menschen appliziert werden. Die Applikation kann dabei systemisch oder topisch erfolgen, bei tierischen Organismen insbesondere parenteral, z.B. durch intravenöse, intraperitoneale, intramuskuläre, intra- oder subkutane Injektion, intravenöse Infusion, pulmonare, buccale, nasale, dermale oder transdermale Applikation. Auch andere Applikationswege, z.B. orale/enterale Applikation sind unter geeigneten Bedingungen und mit entsprechenden Formulierungen möglich. Der Transfektionskomplex kann auch lokal direkt in ein Zielgewebe, etwa ein Organ oder einTumor, appliziert werden. Zur Applikation in vivo sollte der Transfektionskomplex in pharmazeutisch verträglicher Form vorliegen. Er kann dafür mit einem pharmazeutisch akzeptablen Träger kombiniert werden. Die Formulierung richtet sich dabei nach dem Applikationsmodus. Zur Injektion oder Infusion kann der Transfektionskomplex beispielsweise in isotonischer Kochsalzlösung oder in einem isotonischen Puffer wie PBS (phosphate buffered saline) oder Ringer's Lösung vorliegen, zur pulmonaren Verabreichung zum Beispiel in Form eines pharmazeutisch verträglichen Aerosols. Auch feste Formulierungen können je nach Verwendungszweck gewählt werden, beispielsweise gefriergetrocknete Präparate, die vor Verabreichung mit Wasser rekonstituiert werden können. Dem Fachmann sind geeignete pharmazeutische Träger und Formulierungen bekannt, zum Beispiel aus Gennaro (Hrsg.), Remington: The Science and Practice of Pharmacy, 19th Ed., 1995, Mack Publishing Co., Easton, PA, USA. Auf diese Weise kann der Transfektionskomplex bei Verwendung einer entsprechenden Nukleinsäure als Arzneimittel in der Gentherapie eingesetzt werden.

Die Dosierung wählt der Fachmann in Abhängigkeit von der Art der Erkrankung, dem Zustand des Patienten, dem therapeutischen Ansatz sowie weiteren Faktoren. Im allgemeinen wird bei humaner Anwendung die Menge der verabreichten DNA im Bereich von 0.1 µg bis 100 mg/kg Körpergewicht liegen, vorzugsweise 2.5 µg bis 10 mg/kg Körpergewicht.

Eine gentherapeutische Anwendung ist beispielsweise die selektive Transfektion aktivierter vaskulärer Endothelzellen, die eine wichtige Rolle bei Entzündungsprozessen spielen. Durch Einbringen und Expression von geeigneten Genen in diesen Zellen kann die inflammatorische Antwort moduliert werden (Harari et al., supra). Ein zielspezifisches Bindungsmolekül, das für diese Anwendung geeignet ist, ist ein monoklonaler Antikörper gegen das Zelloberflächenantigen CD62E (E-Selectin, ELAM-1), z.B. der monoklonale Antikörper, der von der HybridomaZelllinie H18/7 (ATCC HB-11684) produziert wird. Eine geeignete Nukleinsäure wäre beispielsweise ein Expressionsvektor, der das Gen für das antiinflammatorisch wirkende Zytokin Interleukin-10 (IL-10) exprimieren kann (Henke et al., J Immunol 2000, 164(4):2131-41). Für eine solche Anwendung ist die intravenöse Injektion des Transfektionskomplexes bevorzugt.

Ein anderes klinisches Anwendungsgebiet ist die Gentherapie von Krebserkrankungen. Tumorzellen können mit der vorliegenden Erfindung selektiv transfiziert werden, indem zielspezifische Bindungsmoleküle verwendet werden, die selektiv an spezifische Oberflächenantigene von Tumorzellen binden, zum Beispiel Antikörper gegen CD44v6, das ein Marker für Plattenepithelkarzinome ist (Heider et al., Cancer Immunol Immunother 1996, 43(4):245-53), oder MART-1 (Ribas et al., Cancer Gene Ther 1999, 6:523-36), das ein Markerprotein für Melanome ist. Beispiele für geeignete Nukleinsäuren für solche Anwendungen sind Expressionsvektoren, die die Gene für die Apoptose auslösenden Proteine E2F-1 (Dong et al., Cancer 1999, 86:2021-33) oder Apoptin (Pietersen et al., Gene Ther 1999, 6:882-92) exprimieren können.

Protokolle für die Transfektion von Zellen in vivo durch Applikation von Nukleinsäure/Dendrimer-Komplexen in tierische Organismen sind im Stand der Technik bekannt (Turunen et al., Gene Ther 1999, 6(1):6-11; Maruyama-Tabata et al., Gene Ther 2000, 7(1):53-60; Harada et al., Gene Ther 2000, 7(1):27-36). Die Auswahl von Applikationsart und Dosierung hängt von der Art der Erkrankung, dem Körpergewicht des Patienten usw. ab und liegt im Können des Durchschnittsfachmanns.

Besonders geeignet ist das erfindungsgemäße Verfahren für Zellen, die in Zellkultur in Suspension wachsen (Suspensionszellen), also nicht an Substratoberflächen adhärent wachsen. Insbesondere ist das Verfahren geeignet für Blutzellen oder Blutzeillinien, zum Beispiel B- oder T-Zellen oder davon abgeleitete Zelllinien.

In einem weiteren Aspekt betrifft die vorliegende Erfindung eine Zusammensetzung, enthaltend einen Komplex aus Nukleinsäure und verzweigtem kationischen Polymer, wobei das verzweigte kationische Polymer die Eigenschaft hat, mit Nukleinsäuren Komplexe zu bilden, dadurch gekennzeichnet, daß ein Anteil des verzweigten kationischen Polymers biotinyliert ist. Das kationisches Polymer ist ein Dendrimer wie vorstehend beschrieben.

In einem weiteren Aspekt betrifft die vorliegenden Erfindung eine Zusammensetzung, enthaltend einen Komplex aus Nukleinsäure und Dendrimer, dadurch gekennzeichnet, daß ein Anteil der Dendrimermoleküle biotinyliert ist. Bevorzugt beträgt dabei der Anteil der Dendrimermoleküle, die biotinyliert sind, an der Gesamtmenge der Dendrimermoleküle 0,5 bis 50 % (Mol/Mol), bevorzugt 5 bis 20 % (Mol/Mol), besonders bevorzugt 10 bis 15 % (Mol/Mol).

In einem weiteren Aspekt betrifft die vorliegende Erfindung eine Zusammensetzung, enthaltend einen Komplex aus Nukleinsäure und Dendrimer, dadurch kennzeichnet, daß ein Anteil der Dendrimermoleküle biotinyliert ist und ein Anteil nicht biotinyliert ist herstellbar durch Mischung von biotinyliertem Dendrimer mit nicht-biotinyliertem Dendrimer in einem Verhältnis von von 1:1 bis 1:256 (Gew/Gew), bevorzugt 1: 2 bis 1:128 (Gew/Gew), besonders bevorzugt 1:4 bis 1:24 (Gew/Gew), ganz besonders bevorzugt 1:6 bis 1:10 (Gew/Gew).

Ein weiterer Aspekt der Erfindung ist eine Zusammensetzung, enthaltend einen Komplex aus Nukleinsäure und Dendrimer, wobei ein Anteil der Dendrimermoleküle biotinyliert ist und ein Anteil nicht biotinyliert ist, herstellbar durch ein Verfahren mit den Schritten
(a) Umsetzung von Dendrimer mit einer äquimolaren Menge eines aktivierten Biotins oder Biotinderivats oder einem molaren Überschuß an aktiviertem Biotin oder aktiviertem Biotinderivat, vorzugsweise einem 2- bis 64fachen molaren Überschuß, besonders bevorzugt einem 2- bis 12fachen molaren Überschuß unter Bedingungen, bei denen es zur kovalenten Verknüpfung zwischen Dendrimer und Biotin oder Biotinderivat kommt;
(b) Mischen des unter (a) gebildeten biotinylierten Dendrimers mit nicht-biotinyliertem Dendrimer, vorzugsweise in einem Massenverhältnis von 1:1 bis 1:256, besonders bevorzugt in einem Massenverhältnis von 1:4 bis 1:24;
(c) Inkubation der unter (b) entstandenen Mischung mit Nukleinsäure unter Bedingungen, unter denen sich ein Komplex aus Nukleinsäure und Dendrimer bilden kann.

In einem weiteren Aspekt enthält eine solche Zusammensetzung einen Komplex wie in einem der vorangehenden Absätze definiert, der zusätzlich Avidin oder Streptavidin sowie ein biotinyliertes Ziel-spezifisches Bindungsmolekül enthält. Ein solches zielspezifische Bindungsmolekül bindet vorzugsweise spezifisch an ein Zelloberflächenmolekül, beispielsweise Transferrin oder ein Antikörper.

Ein weiterer Aspekt der Erfindung ist ein Reagenzienkit zum Einbringen von Nukleinsäure in Zellen, mindestens enthaltend
(a) Dendrimer, wobei ein Anteil der Dendrimermoleküle biotinyliert ist und ein Anteil nicht biotinyliert ist, wobei de Anteil der Dendrimer-moleküle, die biotinyliert sind, an der Gesamtmenge der Dendrimer-moleküle 0.5 bis 50% (Mol/Mol) beträgt; und
(b) Avidin oder Streptavidin.

Ein solcher Kit enthält die Komponenten in einer gemeinsamen Verpackungseinheit, gegebenfalls mit zusätzlichen Komponenten und/oder Hitfsmitteln wie Pufferlösungen o.a̅. sowie gegebenenfalls mit einer Arbeitsanleitung und/oder Produktinformation auf Papier, Kunststofffolie oder elektronischem Datenträger. Ein solcher Reagenzienkit kann vorteilhaft für das erfindungsgemäße Verfahren eingesetzt werden. Bevorzugt enthält ein solcher Reagenzienkit als zusätzliche Komponente mindestens ein biotinyliertes zielspezifisches Bindungsmolekül und/oder mindestens eine Nukleinsäure. Ein weiterer Aspekt der vorliegenden Erfindung ist die Verwendung eines solchen Reagenzienkits zum Einbringen von Nukleinsäure in Zellen, insbesondere Suspensionszellen wie T- und B-Zellen.

Ein weiterer Aspekt der vorliegenden Erfindung ist ein Verfahren zur Herstellung eines Komplexes aus Nukleinsäure und Dendrimer, wobei ein Anteil der Dendrimermoleküle biotinyliert ist, mit den Schritten
(a) Umsetzung von Dendrimer mit einer äquimolaren Menge eines aktivierten Biotins oder Biotinderivates oder einem molaren Überschuß an aktiviertem Biotin oder Biotinderivat, vorzugsweise einem 2- bis 64fachen molaren Überschuß, besonders bevorzugt einem 2- bis 12flachen molaren Überschuß unter Bedingungen, bei denen es zur kovalenten Verknüpfung zwischen Dendrimer und Biotin oder Biotinderivat kommt;
(b) Mischen des unter (a) gebildeten biotinylierten Dendrimers mit nicht-biotinyliertem Dendrimer, vorzugsweise in einem Massenverhältnis von 1:1 bis 1:256, besonders bevorzugt in einem Massenverhältnis von 1:4 bis 1:24;
(c) Inkubation der unter (b) entstandenen Mischung mit Nukleinsäure unter Bedingungen, unter denen sich ein Komplex aus Nukleinsäure und Dendrimer bilden kann.

Ein weiterer Aspekt der vorliegenden Erfindung ist eine Zusammensetzung, enthaltend Dendrimer, wobei ein Anteil der Dendrimermoleküle biotinyliert ist, herstellbar nach einem Verfahren mit den Schritten
(a) Umsetzung von Dendrimer mit einer äquimolaren Menge aktivierten Biotins oder Biotinderivats oder einem molaren Überschuß an aktiviertem Biotin oder Biotinderivat, vorzugsweise einem 2- bis 64fachen molaren Überschuß, besonders bevorzugt einem 2- bis 12fachen molaren Überschuß unter Bedingungen, bei denen es zur kovalenten Verknüpfung zwischen Dendrimer und Biotin kommt;
(b) Mischen des unter (a) gebildeten biotinylierten Dendrimers mit nicht-biotinyliertem Dendrimer, vorzugsweise in einem Massenverhältnis von 1:1 bis 1:256, besonders bevorzugt in einem Massenverhältnis von 1:4 bis 1:24.

Ein weiterer Aspekt der vorliegenden Erfindung ist eine pharmazeutische Zusammensetzung, enthaltend ein Dendrimer, wobei ein Anteil der Dendrimermoleküle biotinyliert ist, wie vorstehend beschrieben, sowie gegebenenfalls einen pharmazeutisch akzeptablen Träger.

Ein weiterer Aspekt der vorliegenden Erfindung ist eine pharmazeutische Zusammensetzung, enthaltend einen Komplex aus Nukleinsäure und Dendrimer, wobei ein Anteil der Dendrimermoleküle biotinyliert ist, wie vorstehend beschrieben, sowie gegebenenfalls einen pharmazeutisch akzeptablen Träger.

Ein weiterer Aspekt der vorliegenden Erfindung ist eine pharmazeutische Zusammensetzung, enthaltend einen Komplex aus Nukleinsäure, Dendrimer, Avidin oder Streptavidin, und einem zielspezifischen Bindungsmolekül, wobei das zielspezifische Bindungsmolekül sowie ein Anteil der Dendrimermoleküle biotinyliert ist, wie vorstehend beschrieben, sowie gegebenenfalls einen pharmazeutisch akzeptablen Träger.

Ein weiterer Aspekt der vorliegenden Erfindung ist die Verwendung eines Dendrimers, wobei ein Anteil der Dendrimermoleküle biotinyliert ist, wie vorstehend beschrieben, in der Gentherapie.

Ein weiterer Aspekt der vorliegenden Erfindung ist die Verwendung eines Komplexes aus Nukleinsäure und Dendrimer, wobei ein Anteil der Dendrimermoleküle biotinyliert ist, wie vorstehend beschrieben, in der Gentherapie.

Ein weiterer Aspekt der vorliegenden Erfindung ist die Verwendung eines Komplexes aus Nukleinsäure, Dendrimer, Avidin oder Streptavidin, und einem zielspezifischen Bindungsmolekül, wobei das zielspezifische Bindungsmolekül sowie ein Anteil der Dendrimermoleküle biotinyliert ist, wie vorstehend beschrieben, in der Gentherapie.

Ein weiterer Aspekt der vorliegenden Erfindung ist die Verwendung eines Dendrimers, wobei ein Anteil der Dendrimermoleküle biotinyliert ist, wie vorstehend beschrieben, zur Herstellung eines Arzneimittels für die Gentherapie.

Ein weiterer Aspekt der vorliegenden Erfindung ist die Verwendung eines Komplexes aus Nukleinsäure und Dendrimer, wobei ein Anteil der Dendrimermoleküle biotinyliert ist, wie vorstehend beschrieben, zur Herstellung eines Arzneimittels für die Gentherapie.

Ein weiterer Aspekt der vorliegenden Erfindung ist die Verwendung eines Komplexes aus Nukleinsäure, Dendrimer, Avidin oder Streptavidin, und einem zielspezifischen Bindungsmolekül, wobei das zielspezifische Bindungsmolekül sowie ein Anteil der Dendrimermoleküle biotinyliert ist, wie vorstehend beschrieben, zur Herstellung eines Arzneimittels für die Gentherapie.

Des weiteren beschrieben ist die Verwendung eines Dendrimers, wobei ein Anteil der Dendrimermoleküle biotinyliert ist, wie vorstehend beschrieben, in einem Verfahren zum Einbringen von Nukleinsäure in Zellen in vivo mit den Schritten
(a) Mischen einer Nukleinsäure mit einem Dendrimer, wobei ein Anteil der Dendrimermoleküle biotinyliert ist;
(b) Mischen des entstandenen Komplexes aus Nukleinsäuren und Dendrimer mit einem zweiten Komplex, bestehend aus Avidin oder Streptavidin und einem biotinylierten Ziel-spezifischen Bindungsmolekül;
(c) Verabreichung des in Schritt (b) entstandenen Komplexes in einen pflanzlichen oder tierischen Organismus, gegebenenfalls in Verbindung mit einem pharmazeutisch akzeptablen Träger.

### Abbildungen

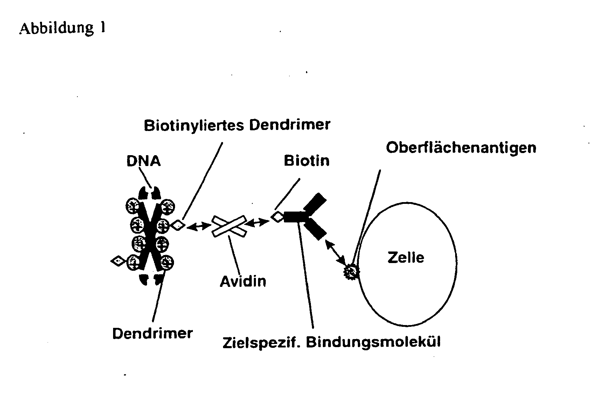

**Abbildung 1****:** Grundprinzip des Avidin-Biotin-Systems.

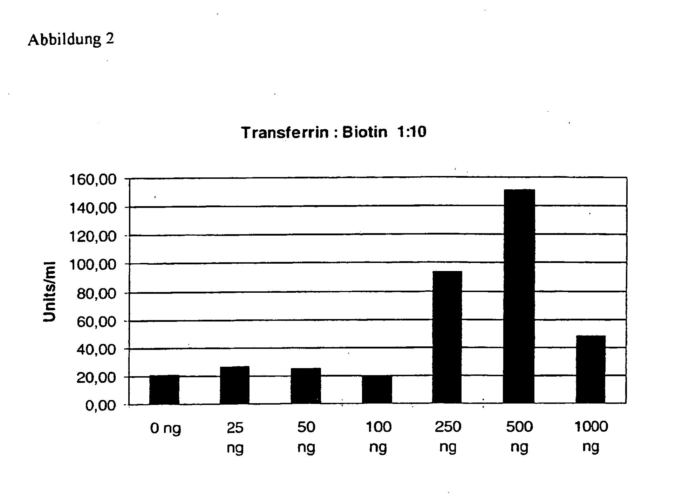

**Abbildung 2****:** Einfluß des Biotinylierungsgrades des Transferrins auf die Transfektionseffizienz. Biotinylierungsgrad 1:10 (Transferrin/Biotin). Die Abszissenwerte geben die im Transfektionsexperiment eingesetzte Menge biotinylierten Transferrins pro Well an. Die Ordinatenwerte geben die ß-Galaktosidase-Aktivität als Maß für die Transfektionseffizienz an. Siehe Beispiel 1.

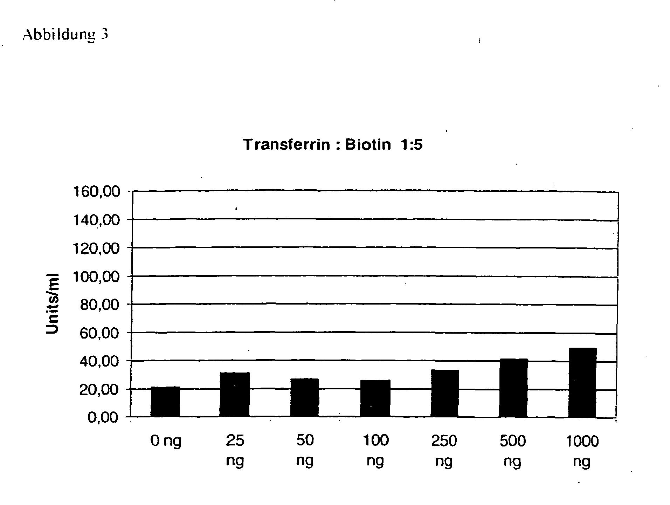

**Abbildung 3****:** Einfluß des Biotinylierungsgrades des Transferrins auf die Transfektionseffizienz. Biotinylierungsgrad 1:5 (Transferrin/Biotin). Die Abszissenwerte geben die im Transfektionsexperiment eingesetzte Menge biotinylierten Transferrins pro Well an. Die Ordinatenwerte geben die ß-Galaktosidase-Aktivität als Maß für die Transfektionseffizienz an. Siehe Beispiel 1.

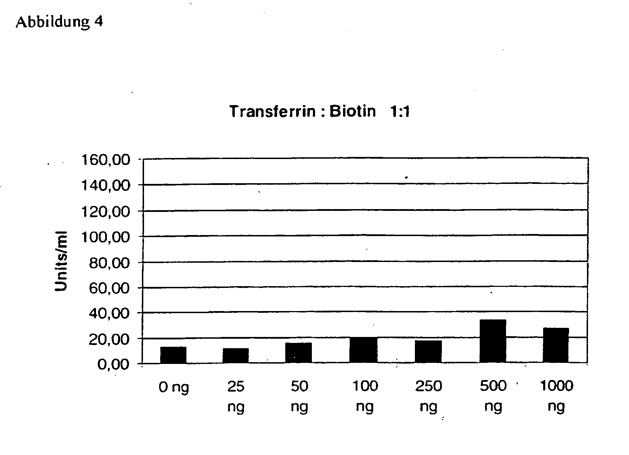

**Abbildung 4****:.** Einfluß des Biotinylierungsgrades des Transferrins auf die Transfektionseffizienz. Biotinylierungsgrad 1:1 (Transferrin/Biotin). Die Abszissenwerte geben die im Transfektionsexperiment eingesetzte Menge biotinylierten Transferrins pro Well an. Die Ordinatenwerte geben die ß-Galaktosidase-Aktivität als Maß für die Transfektionseffizienz an. Siehe Beispiel 1.

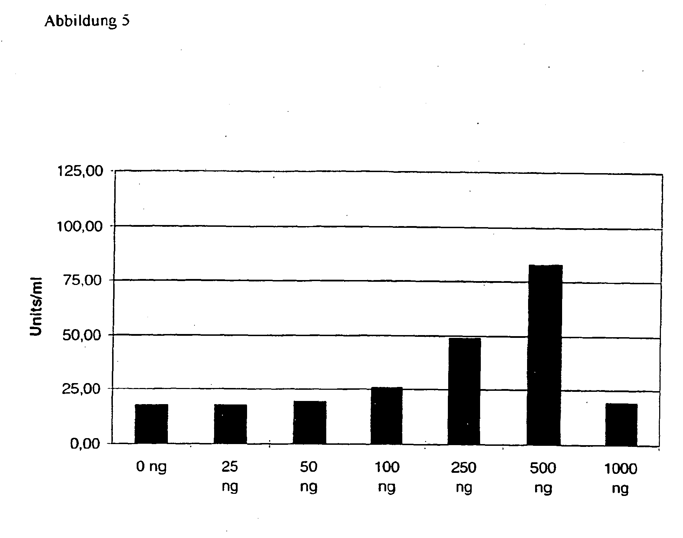

**Abbildung 5****::** Transfektionseffizienz bei unterschiedlicher Reihenfolge der Komplexbildung. Verfahrensschritte: (a) Avidin + biotinyliertes Transferrin; 10 Minuten Inkubation; (b) SuperFect (SF)/ biotinyliertes SF + DNA; 5 Minuten Inkubation; (c) Mischen der beiden Komplexe; 20 Minuten Inkubation. Die Abszissenwerte geben die im Transfektionsexperiment eingesetzte Menge Transferrin pro Well an. Die Ordinatenwerte geben die ß-Galaktosidase-Aktivität als Maß für die Transfektionseffizienz an. Siehe Beispiel 2.

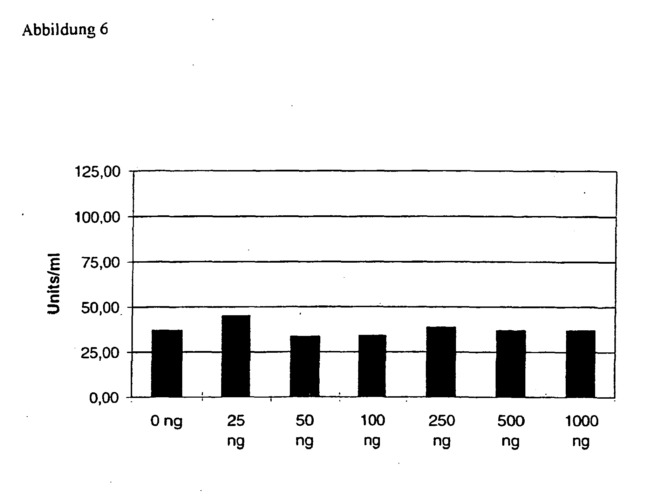

**Abbildung 6****: :** Transfektionseffizienz bei unterschiedlicher Reihenfolge der Komplexbildung. Verfahrensschritte: (a) SF/ biotinyl. SF + DNA; 5 Minuten Inkubation; (b) Zugabe von Avidin; 10 Minuten Inkubation; (c) Zugabe von biotinyl. Transferrin; 20 Minuten Inkubation. Die Abszissenwerte geben die im Transfektionsexperiment eingesetzte Menge Transferrin pro Well an. Die Ordinatenwerte geben die β-Galaktosidase-Aktivität als Maß für die Transfektionseffizienz an. Siehe Beispiel 2.

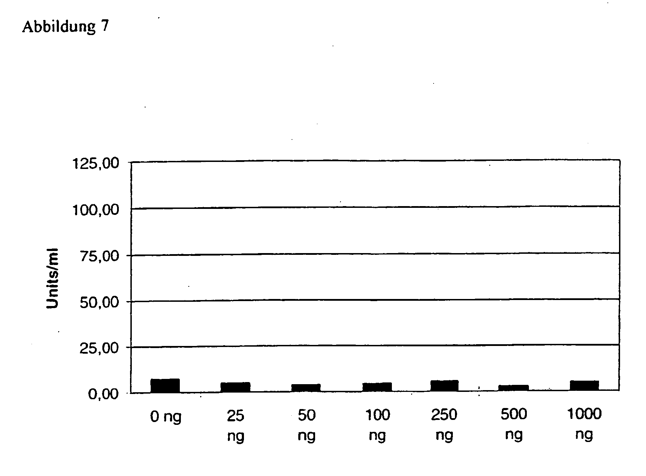

**Abbildung 7****:** Transfektionseffizienz bei unterschiedlicher Reihenfolge der Komplexbildung. Verfahrensschritte: (a) Biotinyl. SF + Avidin; 10 Minuten Inkubation; (b) Zugabe von SuperFect; Mischen; (c) Zugabe der DNA; 5 Minuten Inkubation; (d) Zugabe von biotinyl. Transferrin; 30 Minuten Inkubation. Die Abszissenwerte geben die im Transfektionsexperiment eingesetzte Menge Transferrin pro Well an. Die Ordinatenwerte geben die ß-Galaktosidase-Aktivität als Maß für die Transfektionseffizienz an. Siehe Beispiel 2.

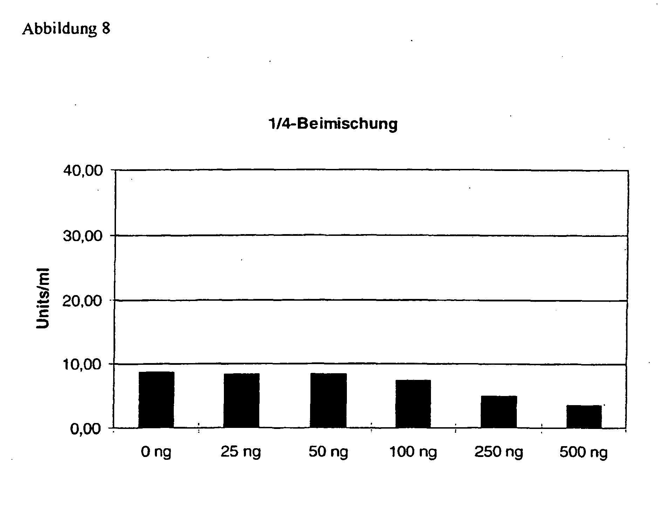

**Abbildung 8****:** Transfektionseffizienz bei direkter Kopplung von Avidin an SuperFect. ¼ Beimischung Avidin-SF-Kopplungsprodukt zur Gesamtmenge an Superfect. Die Abszissenwerte geben die im Transfektionsexperiment eingesetzte Menge an biotinyliertem Transferrin pro Well an. Die Ordinatenwerte geben die β-Galaktosidase-Aktivität als Maß für die Transfektionseffizienz an. Siehe Beispiel 3.

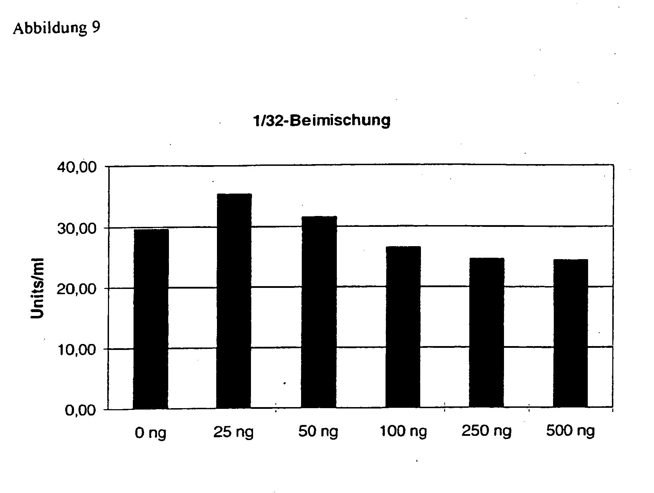

**Abbildung 9****:** Transfektionseffizienz bei direkter Kopplung von Avidin an SuperFect. 1/32 Beimischung Avidin-SF-Kopplungsprodukt zur Gesamtmenge an Superfect. Die Abszissenwerte geben die im Transfektionsexperiment eingesetzte Menge an biotinyliertem Transferrin pro Well an. Die Ordinatenwerte geben die β-Galaktosidase-Aktivität als Maß für die Transfektionseffizienz an. Siehe Beispiel 3.

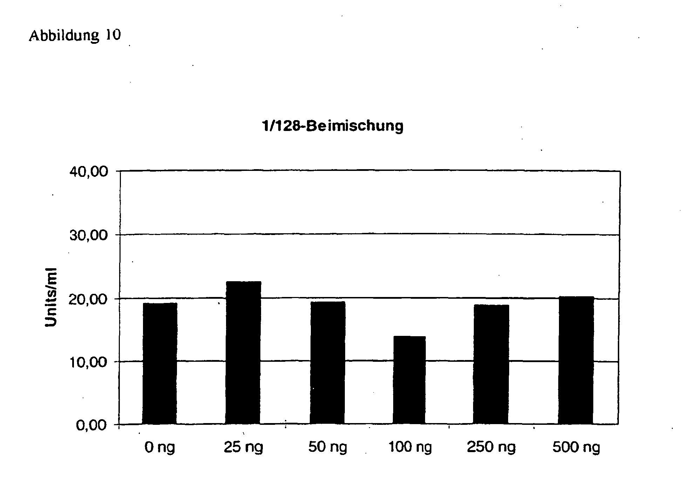

**Abbildung 10****:** Transfektionseffizienz bei direkter Kopplung von Avidin an SuperFect. 1/128 Beimischung Avidin-SF-Kopplungsprodukt zur Gesamtmenge an Superfect. Die Abszissenwerte geben die im Transfektionsexperiment eingesetzte Menge an biotinyliertem Transferrin pro Well an. Die Ordinatenwerte geben die β-Galaktosidase-Aktivität als Maß für die Transfektionseffizienz an. Siehe Beispiel 3.

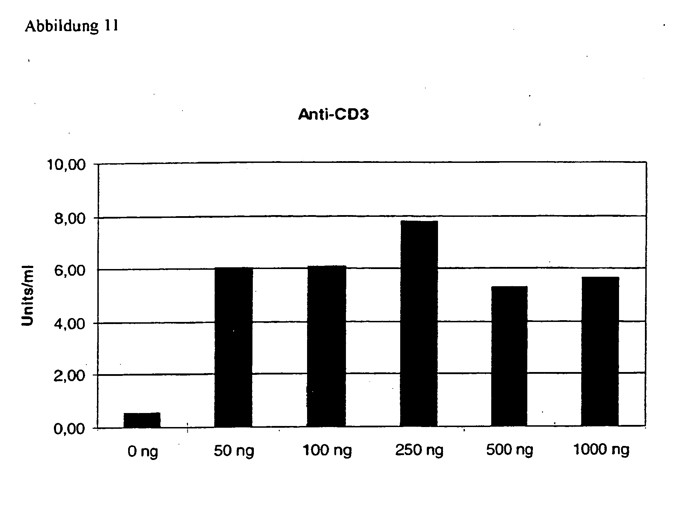

**Abbildung 11****:** Transfektionseffizienz der Transfektion von Jurkat-Zellen mit biotinyliertem Anti-CD3-Antikörper. Die Abszissenwerte geben die im Transfektionsexperiment eingesetzte Menge an biotinyliertem Antikörper an. Die Ordinatenwerte geben die ß-Galaktosidase-Aktivität als Maß für die Transfektionseffizienz an. Siehe Beispiel 4.

### Beispiele

### Beispiel 1: Gezielte Transfektion von K562-Zellen mit biotinyliertem Transferrin/ unterschiedliche Biotinylierungsstufen des Transferrins

### Biotinylierungsreaktionen

Humanes Holo-Transferrin wurde von Calbiochem (La Jolla, CA, USA) bezogen, und in PBS gelöst. Die Biotinylierung erfolgte mit D-Biotinoyl-ε-aminocapronsäure-N-hydroxysuccinimidester (Biotin-7-NHS) mittels eines kommerziell erhältlichen Biotin Protein Labeling Kits (Roche Molecular Biochemicals, Hoffman-LaRoche AG, Grenzach-Wyhlen, Deutschland) gemäß Herstellerangaben. Der Biotinylierungsgrad wurde mittels HABA (4' Hydroxyazobenzen-2-Carboxylsäure)-Farbstoffreaktion (Fluka, Milwaukee, WI, USA) und Standard-Proteinbestimmung gemessen (Hermanson, supra, Seiten 591-592).

Die Biotinylierung von Dendrimer (SuperFect^{®}, Qiagen GmbH, Hilden, Deutschland), einem aktivierten TAEA-PAMAM der 6. Generation, erfolgte mit N-(+)-Biotinyl-6-aminocapronsäure-N-succinimidylester gemäß Hermanson, supra, Seiten 575 bis 580. Dabei wurden jeweils 4 mg Superfect mit dem entsprechenden molaren Überschuß an Biotin (4-, 8-, 16- oder 32fach) umgesetzt. Anschließend erfolgte eine Dialyse.

### Kultivierung der Suspensionszellen:

Die Zellinie K562 (ATCC CCL-243) wurde zunächst drei Tage in Spinner-Flaschen in RPMI-Medium mit 10 % FCS kultiviert. Die Zelldichte zu Beginn betrug 1,5x10⁵ bis 2x10⁵ Zellen pro ml. Nach drei Tagen wurde die Kultur auf eine Dichte von ca. 3x10⁵ Zellen pro ml verdünnt. Nach weiteren 24 Stunden Kultivierung wurden die Zellen in 96-Well-Platten à 30 000 K562-Zellen pro Well in 100 µl ausgesät. Erfolgte am darauffolgenden Tag nochmals eine Transfektion, so wurde die Spinner-Kultur zuvor auf eine Dichte von ca. 3x10⁵ Zellen pro ml verdünnt.

### Transfektion:

1. Pro Fünffachansatz (der anschließend für vier Wells einer 96-Well-Platte benutzt wurde) wurden 10 µl einer Transferrinlösung (verdünnt in Medium ohne Serum) mit 10 µl einer Avidinlösung (Fluka, Milwaukee, WI, USA; verdünnt in Medium ohne Serum) gemischt und 10 Minuten bei Raumtemperatur inkubiert. Es wurden umgerechnet 1 µg Avidin und 25 ng bis 1 µg biotinyliertes Transferrin pro Well eingesetzt.
2. Zur Komplexbildung wurden 150 µl DNA (pCMVβ, Clontech Laboratories, Inc., Palo Alto, CA, USA; ein Expressionsplasmid für ß-Galaktosidase)-Lösung mit 100 µl Reagenzlösung (SuperFect-Reagenz; Mischung aus biotinyliertem und nicht-biotinyliertem SuperFect im Verhältnis 1:8 ; das biotinylierte SuperFect wurde mit einem 4fachen molaren Überschuß an Biotinreagenz hergestellt) für fünf Minuten bei Raumtemperatur inkubiert. Die eingesetzten Mengen entsprachen dabei 0,5 µg DNA und 1,5 µg Transfektionsreagenz pro Well.
3. Die Komplexe aus (1) und (2) wurden miteinander gemischt und 30 Minuten bei Raumtemperatur inkubiert.
4. Je 50 µl der Komplexe aus (3) wurden pro Well zu den Zellen zugegeben und mit der Zellsuspension gemischt und 48 h inkubiert.
5. Vor der Lysis der Zellen wurden diese in Mikrotiterplatten mit konischen Böden überführt. In diesen Mikrotiterplatten können die Zellen durch Zentrifugieren für fünf Minuten bei 2000 UpM pelletiert werden. Vor Aufnahme in Lysispuffer (5 mM MgCl₂, 1% NP-40, 100 mM NaCl, 10 mM Tris/HCl, pH 7,4) und Zurücküberführen in die ursprünglichen Platten wurden die Zellen und die ursprünglichen Wells zweimal mit PBS gewaschen.
6. Im Anschluß erfolgte zur Bestimmung der Transfektionseffizienz ein β-Galaktosidase-Assay mit ONPG (2-Nitrophenyl-β-D-galaktopyranosid, Merck KGaA, Darmstadt, Deutschland) als Substrat bzw. eine *X-*Gal-*In*-*Situ* -Färbung (Ausubel et al. (Hrsg.), Current Protocols in Molecular Biology, Wiley & Sons, New York 1988, 9.5.12).

Entsprechend des Protokolls für die gezielte Transfektion von Suspensionszellen wurden K562-Zellen im 96-Well Maßstab transfiziert. Es wurden Transferrine eingesetzt, für die Biotinylierungsgrade von durchschnittlich ca. 10, 5 oder ein Biotinmolekül pro Transferrinmolekül gemessen worden waren. Bei einem Biotinylierungsgrad von durchschnittlich ca. 10 Biotinen pro Transferrinmolekül kommt es bei einer eingesetzten Menge von 250 ng bis 1000 ng Transferrin pro Well zu einer Erhöhung der Transfektionseffizienz um ca. den Faktor 7,5. Für die Biotinylierungsgrade 1:5 und 1:1 kam es nur zu einer schwachen Erhöhung um das maximal zweifache (siehe Abbildungen 2 bis 4). Ein höherer Biotinylierungsgrad (1:13) zeigte einen geringeren Effekt auf die Transfektionseffizienz. In einem weiteren Experiment wurde für 1:10 biotinyliertes Transferrin mittels X-Gal-*In*-*Situ*-Färbung der Prozentsatz transfizierter Zellen bestimmt. Während die ß-Galaktosidase-Aktivität von 12,2 Units/ml (ohne Transferrin) auf 71,7 Units/ml (500 ng biotinyliertes Transferrin) gesteigert werden konnte, wurde für 500 ng biotinyliertes Transferrin eine Transfektionseffizienz von 28 % transfizierten Zellen ermittelt.

Es wurde ferner der Einfluß des Biotinylierungsgrades des SuperFect auf die Transfektion untersucht. Eingesetzt wurde biotinyliertes SuperFect, bei dem in der Biotinylierungsreaktion ein vier-, acht-, 16- oder 32facher Überschuß an Biotin eingesetzt worden war (im Folgenden als 1:4, 1:8, 1:16 und 1:32 biotinyliert bezeichnet. Pro Well wurden 0,5 µg DNA (pCMVß; ein Expressionsplasmid für β-Galaktosidase) und 1,5 µg SuperFect-Reagenz zur Transfektion verwendet. Es wurde zwischen der Hälfte (1/2) und 1/128 an biotinyliertem SuperFect zugegeben. Mit zunehmender Menge an biotinyliertem SuperFect nimmt die Transfektionseffizienz ab.

Es wurden außerdem für alle vier unterschiedlich biotinylierten SuperFect-Ansätze Experimente durchgeführt, bei denen steigende Mengen an Avidin zu den SuperFect-biotinyliertes SuperFect-DNA-Komplexen zugegeben wurden. Es wurden zwischen 10 ng und 5000 ng Avidin pro Well (einer 96-Well-Platte) in einem Volumen von 10 µl pro Fünffachansatz eingesetzt. Die Inkubation mit Avidin erfolgte für 30 Minuten bei Raumtemperatur. Mit steigender Menge an Avidin kommt es zuerst zu einer Zunahme der Transfektionseffizienz, die als ß-Galaktosidase-Aktivität gemessen werden kann. Bei einem Anteil von 1/8 biotinyliertem SuperFect kam es über 1000 ng Avidin pro Well zu einer Abnahme der β-Gal-Aktivität. Für weitere Experimente wurden 1:4 biotinyliertes SuperFect in der Beimischung 1/8 und 1000 ng Avidin pro Well verwendet.

Die Verwendung von biotinyliertem SuperFect in Kombination mit Avidin und biotinyliertem Transferrin (Verhältnis von 10 Molekülen Biotin pro 1 Molekül Transferrin) ermöglicht es, in K562-Zellen die Transfektionseffizienz von 2 bis 5 % bis auf über 25% transfizierte Zellen zu erhöhen.

### Beispiel 2: Transfektionseffizienz in Abhängigkeit von der Reihenfolge der Verfahrensschritte bei der Bildung des Transfektionskomplexes

Um abzuklären, ob die Reihenfolge der Zugabe der einzelnen Komponenten zu den Komplexen auf die Erhöhung der Transfektionseffizienz Einfluß hat, wurden Transfektionsexperimente in K562-Zellen durchgeführt, bei denen nachfolgende Abläufe eingehalten wurden:
a.)
   1. Avidin + biotinyliertes Transferrin; 10 Minuten Inkubation
   2. SuperFect (SF)/ biotinyliertes SF + DNA; 5 Minuten Inkubation
   3. Mischen der beiden Komplexe; 20 Minuten Inkubation
b.)
   1. SF/ biotinyl. SF + DNA; 5 Minuten Inkubation
   2. Zugabe von Avidin; 10 Minuten Inkubation
   3. Zugabe von biotinyl. Transferrin; 20 Minuten Inkubation
c.)
   1. Biotinyl. SF + Avidin; 10 Minuten Inkubation
   2. Zugabe von SuperFect; Mischen
   3. Zugabe der DNA; 5 Minuten Inkubation
   4. Zugabe von biotinyl. Transferrin; 30 Minuten Inkubation

Die Transfektionseffizienz für diese drei Varianten ist in den Abbildungen 5, 6 und 7 dargestellt.

Der Effekt einer Verstärkung der Transfektionseffizienz durch das Biotin-Avidin-System tritt nur auf, wenn DNA und SuperFect/ biotinyl. SuperFect sowie Avidin und biotinyl. Transferrin getrennt voneinander komplexiert und erst anschließend beide Komplexe gemischt werden. Bei Vorinkubation des biotinylierten Superfects mit dem Avidin kommt es sogar zu einem massiven Verlust an Transfektionseffizienz, da wahrscheinlich die Ausbildung des SuperFect-DNA-Komplexes verhindert wird.

### Beispiel 3: Direkte Kopplung von Avidin an SuperFect

Als Alternative zu einem System, bei dem biotinyliertes SuperFect, Avidin und biotinyliertes Transferrin zum Einsatz kommen, wurde in K562-Zellen ein System getestet, das auf eine direkte, chemische Kopplung von Avidin an SuperFect basiert. Die Kopplung wurde gemäß Hermanson, supra, Seiten 575-583 ausgeführt.

Untersucht wurden Avidin-SuperFect-Kopplungsprodukte, bei denen die Kopplung für 2 oder 24 Stunden erfolgte oder ein bidirektionaler Linker (N-Succinimidyl-3-(2-Pyridyldithio)-Propionat) eingesetzt worden war. In den Abbildung 8, 9 und 10 sind beispielhaft die Ergebnisse für ein Avidin-SuperFect-Kopplungsprodukt von zwei Stunden mit folgendem Ablauf der Inkubationsschritte dargestellt:
1. 10 µl einer SuperFect-Avidin-Kopplungsprodukt-Lösung + 10 µl einer Lösung mit biotinyliertem Transferrin (1:10 biotinyliert; die Konzentrationen wurden so gewählt, daß das jeweils gewünschte Endverhältnis (1:4, 1:32, 1:128 (Gew/Gew) Transferrin/Kopplungsprodukt) so wie eine Endkonzentration von 1,5 µg SuperFect/Well erreicht wurde); 15 Minuten Inkubation bei Raumtemperatur;
2. Zugabe von 90 µl SuperFect-Verdünnung; 10 Minuten Inkubation bei Raumtemperatur;
3. Zugabe von 150 µl DNA-Lösung (pCMVß); 10 Minuten Inkubation bei Raumtemperatur.

Alle Verdünnungen wurden in Medium ohne Serum hergestellt; pro Well einer 96-Well-Platte wurden 0,5 µg DNA, 1,5 µg SuperFect-Reagenz mit wechselndem Anteil an Avidin-SF-Kopplungsprodukt (siehe Abbildungen) eingesetzt.)

Angegeben sind auf der Abszisse die eingesetzten Mengen an biotinyliertem Transferrin pro Well und in den Titeln der Diagramme der Anteil an Avidin-SF-Kopplungsprodukt an der SuperFect-Gesamtmenge. Das Ergebnis für eine parallel durchgeführte konventionelle SuperFect-Transfektion ergab 10,3 Units/ml.

Abhängig von der eingesetzten Menge an Avidin-SuperFect-Kopplungsprodukt kommt es zu einer Erhöhung der Transfektionseffizienz (1/32- und 1/128-Beimischung). Durch biotinyliertes Transferrin wird aber in keinem Fall eine weitere Erhöhung der Effizienz bewirkt. Dieses Ergebnis wurde auch bei einer geänderten Reihenfolge der Inkubationen (1. Mischen SF mit Avidin-SF-Kopplungsprodukt; 2. Zugabe der DNA; 3. Zugabe des biotinylierten Transferrins) und der Verwendung alternativer Kopplungsprodukte (Kopplung über 24 Stunden; Verwendung eines bidirektionalen Linkers) erhalten.

### Beispiel 4: Gezielte Transfektion von Jurkat-Zellen mit biotinyliertem anti CD3-Antikörper

### Antikörper

Als Antikörper wurde der kommerziell erhältliche biotinylierte αhumanCD3 von Sigma (Sigma-Aldrich Chemie GmbH, Deisenhofen, Deutschland) verwendet.

### Kultivierung der Suspensionszellen:

Die Zellinie Jurkat (DSMZ ACC 282) wurde zunächst drei Tage in Spinner-Flaschen in RPMI-Medium mit 10 % FCS kultiviert. Die Zelldichte zu Beginn betrug 1,5x10⁵ bis 2x10⁵ Zellen pro ml. Nach drei Tagen wurde die Kultur auf eine Dichte von ca. 3x10⁵ Zellen pro ml verdünnt. Nach weiteren 24 Stunden Kultivierung wurden die Zellen in 96-Well-Platten ä 50 000 Zellen pro Well in 100 µl ausgesät. Erfolgte am darauffolgenden Tag nochmals eine Transfektion, so wurde die Spinner-Kultur zuvor auf eine Dichte von ca. 3x10⁵ Zellen pro ml verdünnt.

### Transfektion:

1. Pro Fünffachansatz (der anschließend für vier Wells einer 96-Well-Platte benutzt wurde) wurden 10 µl einer Antikörperlösung (verdünnt in Medium ohne Serum) mit 10 µl einer Avidinlösung (verdünnt in Medium ohne Serum) gemischt und 10 Minuten bei Raumtemperatur inkubiert. Es wurden umgerechnet 0,75 µg Avidin und 25 ng bis 1 µg biotinylierter Antikörper pro Well eingesetzt.
2. Zur Komplexbildung wurden 150 µl DNA (pCMVß; ein Expressionsplasmid für β-Galaktosidase)-Lösung mit 100 µl Reagenzlösung (SuperFect-Reagenz; Mischung aus biotinyliertem und nicht-biotinyliertem SuperFect im Verhältnis 1:8 ; das biotinylierte SuperFect wurde mit einem 4fachen molaren Überschuß an Biotinreagenz hergestellt) für fünf Minuten bei Raumtemperatur inkubiert. Die eingesetzten Mengen entsprachen dabei 0,5 µg DNA und 1,5 µg Transfektionsreagenz pro Well.
3. Die Komplexe aus (1) und (2) wurden miteinander gemischt und 30 Minuten bei Raumtemperatur inkubiert.
4. Je 50 µl der Komplexe aus (3) wurden pro Well zu den Zellen zugegeben und mit der Zellsuspension gemischt und für 48 h inkubiert.
5. Vor der Lysis der Zellen wurden diese in Mikrotiterplatten mit konischen Böden überführt. In diesen Mikrotiterplatten können die Zellen durch Zentrifugieren für fünf Minuten bei 2000 UpM pelletiert werden. Vor Aufnahme in Lysispuffer und Zurücküberführen in die ursprünglichen Platten wurden die Zellen und die ursprünglichen Wells zweimal mit PBS gewaschen.
6. Im Anschluß erfolgte zur Bestimmung der Transfektionseffizienz ein β-Galaktosidase-Assay mit ONPG als Substrat bzw. eine X-Gal-*In*-*Situ* -Färbung

In dem hier dargestellten Experiment wurde die T-Zellinie Jurkat mit biotiriyliertem anti-CD3-Antikörper transfiziert. Nach Optimierung des Systems wurden 0,75 µg Avidin pro Well und 1:4 biotinyliertes SuperFect in einer Beimischung von 1/8 eingesetzt. Die Menge an biotinyliertem Antikörper ist unter den Balken im Diagramm in Abbildung 11 angegeben. Der Wert für eine Kontrolltransfektion mit SuperFect (ohne Avidin, biotinyliertes SuperFect) lag bei 0,9 Units/ml.

Für 250 ng und 500 ng Antikörper wurde mittels X-Gal-Staining der Prozentsatz an transfizierten Zellen bestimmt; er lag bei 4,8 % (250 ng) bzw. 5,0 % (500 ng) der Zellen. Dieses Ergebnis ließ sich in weiteren Experimenten bestätigen.

## Patentansprüche

1. Zusammensetzung zur Transfektion von Zellen, enthaltend einen Komplex aus Nukleinsäure und einem Dendrimer, wobei ein Anteil der Dendrimermoleküle biotinyliert und ein Anteil der Dendrimermoleküle nicht biotinyliert ist, **dadurch gekennzeichnet, dass** der Anteil der Dendrimermoleküle, die biotinyliert sind, an der Gesamtmenge der Dendrimermoleküle 0,5 bis 50% (Mol/Mol) beträgt.

2. Zusammensetzung nach Anspruch 1, **dadurch gekennzeichnet, daß** der Anteil der Dendrimermoleküle, die biotinyliert sind, an der Gesamtmenge der Dendrimermoleküle 5 bis 20 % (Mol/Mol) beträgt.

3. Zusammensetzung nach einem der vorangehenden Ansprüche, herstellbar durch ein Verfahren mit den Schritten
(a) Umsetzung von Dendrimers mit einer äquimolaren Menge aktivierten Biotins oder eines aktivierten Biotinderivats oder einem molaren Überschuß an aktiviertem Biotin oder aktivierten Biotinderivats, unter Bedingungen, bei denen es zur kovalenten Verknüpfung zwischen Dendrimer und Biotin kommt;
(b) Mischen des unter (a) gebildeten biotinylierten Dendrimers mit nicht-biotinyliertem Dendrimer;
(c) Inkubation der unter (b) entstandenen Mischung mit Nukleinsäure unter Bedingungen, unter denen sich ein Komplex aus Nukleinsäure und Dendrimer bilden kann.

4. Zusammensetzung nach Anspruch 3, wobei der molare Überschuß an aktiviertem Biotin oder aktivierten Biotinderivats ein 2- bis 64facher molarer Überschuß ist.

5. Zusammensetzung nach einem der Ansprüche 3 oder 4, wobei der molare Überschuß an aktiviertem Biotin oder aktivierten Biotinderivats ein 2- bis 12facher molarer Überschuß ist.

6. Zusammensetzung nach einem der Ansprüche 3 bis 5, wobei das Mischen des biotinylierten Dendrimers mit nicht-biotinyliertem Dendrimer in einem Massenverhältnis von 1:1 bis 1:256 erfolgt.

7. Zusammensetzung nach einem der Ansprüche 3 bis 6, wobei das Mischen des biotinylierten Dendrimers mit nicht-biotinyliertem Dendrimer in einem Massenverhältnis von 1:4 bis 1:24 erfolgt.

8. Zusammensetzung nach einem der vorangehenden Ansprüche, **dadurch gekennzeichnet, daß** der Komplex zusätzlich Avidin oder Streptavidin sowie ein biotinyliertes zielspezifisches Bindungsmolekül enthält.

9. Zusammensetzung nach einem der vorangehenden Ansprüche, **dadurch gekennzeichnet, daß** das zielspezifische Bindungsmolekül ein Zelloberflächenmolekül erkennt.

10. Zusammensetzung nach einem der vorangehenden Ansprüche, **dadurch gekennzeichnet, daß** das zielspezifische Bindungsmolekül Transferrin oder ein Antikörper ist.

11. Verfahren zum Einbringen von Nukleinsäure in Zellen *in vitro* mit den Schritten
(a) Mischen einer Nukleinsäure mit einem Dendrimer, wobei ein Anteil der Dendrimermoleküle biotinyliert ist und der Anteil der Dendrimermoleküle, die biotinyliert sind, an der Gesamtmenge der Dendrimermoleküle 5 bis 20% (Mol/Mol) beträgt;
(b) Mischen des entstandenen Komplexes aus Nukleinsäure und Dendrimer mit einem zweiten Komplex, bestehend aus Avidin oder Streptavidin und einem biotinylierten zielspezifischen Bindungsmolekül;
(c) Inkubation des in Schritt (b) entstandenen Komplexes mit Zellen.

12. Verfahren nach Anspruch 11, **dadurch gekennzeichnet, daß** die Zellen in Zellkultur in Suspension wachsen.

13. Verfahren nach einem der Ansprüche 11 oder 12, **dadurch gekennzeichnet, daß** die Zellen Blutzellen oder Blutzelllinien sind.

14. Verfahren nach Anspruch 13, **dadurch gekennzeichnet, dass** die Zellen B- oder T-Zellen oder davon abgeleitete Zelllinien sind.

15. Reagenzienkit zum Einbringen von Nukleinsäure in Zellen, mindestens enthaltend
(a) Dendrimere, wobei ein Anteil der Dendrimermoleküle biotinyliert und ein Anteil der Dendrimermoleküle nicht biotinyliert ist, wobei der Anteil der Dendrimermoleküle, die biotinyliert sind, an der Gesamtmenge der Dendrimermoleküle 0,5 bis 50% (Mol/Mol) beträgt; und
(b) Avidin oder Streptavidin.

16. Reagenzienkit nach Anspruch 15, **dadurch gekennzeichnet, dass** es zusätzlich ein biotinyliertes zielspezifisches Bindungsmolekül und/oder eine Nukleinsäure enthält.

17. Verwendung eines Reagenzienkits nach einem der Ansprüche 15 oder 16 zum Einbringen von Nukleinsäure in Zellen.

18. Verwendung nach Anspruch 17, **dadurch gekennzeichnet, dass** die Zellen in Zellkultur in Suspension wachsen.

19. Verfahren zur Herstellung eines Komplexes aus Nukleinsäure und Dendrimer, wobei ein Anteil der Dendrimermoleküle biotinyliert ist und wobei der Anteil der Dendrimermoleküle, die biotinyliert sind, an der Gesamtmenge der Dendrimermoleküle 0,5 bis 50% (Mol/Mol) beträgt, mit den Schritten
(a) Umsetzung von Dendrimer mit einer äquimolaren Menge aktivierten Biotins oder eines aktivierten Biotinderivats oder einem molaren Überschuß an aktiviertem Biotin oder aktiviertem Biotinderivat, unter Bedingungen, bei denen es zur kovalenten Verknüpfung zwischen Dendrimer und Biotin kommt;
(b) Mischen des unter (a) gebildeten biotinylierten Dendrimers mit nicht-biotinyliertem Dendrimer;
(c) Inkubation der unter (b) entstandenen Mischung mit Nukleinsäure unter Bedingungen, unter denen sich ein Komplex aus Nukleinsäure und Dendrimer bilden kann.

20. Verfahren nach Anspruch 19, wobei der molare Überschuß an aktiviertem Biotin oder aktivierten Biotinderivats ein 2- bis 64facher molarer Überschuß ist.

21. Verfahren nach einem der Ansprüche 19 oder 20, wobei der molare Überschuß an aktiviertem Biotin oder aktivierten Biotinderivats ein 2- bis 12facher molarer Überschuß ist.

22. Verfahren nach einem der Ansprüche 19 bis 21, wobei das Mischen des biotinylierten Dendrimers mit nicht-biotinyliertem Dendrimer in einem Massenverhältnis von 1:1 bis 1:256 erfolgt.

23. Verfahren nach einem der Ansprüche 19 bis 22, wobei das Mischen des biotinylierten Dendrimers mit nicht-biotinyliertem Dendrimer in einem Massenverhältnis von 1:4 bis 1:24 erfolgt.

24. Verfahren zur Herstellung eines Dendrimergemisches, wobei ein Anteil der Dendrimermoleküle biotinyliert ist und wobei der Anteil der Dendrimermoleküle, die biotinyliert sind, an der Gesamtmenge der Dendrimermoleküle 0,5 bis 50% (Mol/Mol) beträgt, mit den Schritten
(a) Umsetzung von Dendrimer mit einer äquimolaren Menge aktivierten Biotins oder eines aktivierten Biotinderivats oder einem molaren Überschuß an aktiviertem Biotin oder aktiviertem Biotinderivat, unter Bedingungen, bei denen es zur kovalenten Verknüpfung zwischen Dendrimer und Biotin kommt;
(b) Mischen des unter (a) gebildeten biotinylierten Dendrimers mit nicht-biotinyliertem Dendrimer.

25. Verfahren nach Anspruch 24, wobei der molare Überschuß an aktiviertem Biotin oder aktivierten Biotinderivats ein 2- bis 64facher molarer Überschuß ist.

26. Verfahren nach einem der Ansprüche 24 oder 25, wobei der molare Überschuß an aktiviertem Biotin oder aktivierten Biotinderivats ein 2- bis 12facher molarer Überschuß ist.

27. Verfahren nach einem der Ansprüche 24 bis 26, wobei das Mischen des biotinylierten Dendrimers mit nicht-biotinyliertem Dendrimer in einem Massenverhältnis von 1:1 bis 1:256 erfolgt.

28. Verfahren nach einem der Ansprüche 24 bis 27, wobei das Mischen des biotinylierten Dendrimers mit nicht-biotinyliertem Dendrimer in einem Massenverhältnis von 1:4 bis 1:24 erfolgt.

29. Dendrimer, wobei ein Anteil der Dendrimermoleküle biotinyliert ist, herstellbar nach einem Verfahren gemäß einem der Ansprüche 24 bis 28.

30. Pharmazeutische Zusammensetzung, enthaltend eine Zusammensetzung nach einem der Ansprüche 1 bis 10 oder ein Dendrimer nach Anspruch 29 sowie gegebenenfalls einen pharmazeutisch akzeptablen Träger.

31. Verwendung einer Zusammensetzung nach einem der Ansprüche 1 bis 10 oder einem Dendrimer nach Anspruch 29 zur Herstellung eines Arzneimittels für die Gentherapie.

## Claims

1. Composition containing a complex of nucleic acid and a dendrimer for transfecting cells, wherein a proportion of the dendrimer molecules is biotinylated and a proportion of the dendrimer molecules is not biotinylated, **characterized in that** the proportion of the dendrimer molecules that are biotinylated is 0.5 to 50% (mol/mol) of the total amount of the dendrimer molecules.

2. Composition according to claim 1, **characterized in that** the proportion of the dendrimer molecules that are biotinylated is 5 to 20% (mol/mol) of the total amount of the dendrimer molecules.

3. Composition according to any one of the preceding claims, obtainable by a method comprising the steps of
(a) reacting dendrimer with an equimolar amount of activated biotin or of an activated biotin derivative or a molar excess of activated biotin or activated biotin derivative under conditions in which covalent bonding takes place between dendrimer and biotin;
(b) mixing the biotinylated dendrimer of step (a) with non-biotinylated dendrimer;
(c) incubation of the mixture formed in step (b) with nucleic acid under conditions in which a complex of nucleic acid and dendrimer can be formed.

4. Composition according to claim 3, wherein the molar excess of activated biotin or activated biotin derivative is a 2- to 64-fold molar excess.

5. Composition according to any one of claims 3 or 4, wherein the molar excess of activated biotin or activated biotin derivative is a 2- to 12-fold molar excess.

6. Composition according to any one of claims 3 to 5, wherein the mixing of the biotinylated dendrimer with non-biotinylated dendrimer is effected in a mass ratio of 1:1 to 1:256.

7. Composition according to any one of claims 3 to 6, wherein the mixing of the biotinylated dendrimer with non-biotinylated dendrimer is effected in a mass ratio of 1:4 to 1:24.

8. Composition according to any one of the preceding claims, **characterized in that** the complex additionally contains avidin or streptavidin and a biotinylated target-specific binding molecule.

9. Composition according to any one of the preceding claims, **characterized in that** the target-specific binding molecule recognizes a cell surface molecule.

10. Composition according to any one of the preceding claims, **characterized in that** the target-specific binding molecule is transferrin or an antibody.

11. Method for introducing nucleic acid into cells *in vitro* comprising the steps of
(a) mixing a nucleic acid with a dendrimer, wherein a proportion of the dendrimer molecules is biotinylated and wherein the proportion of the dendrimer molecules that is biotinylated consists of 5 to 20% (mol/mol) of the total amount of dendrimer molecules;
(b) mixing the resulting complex of nucleic acid and dendrimer with a second complex consisting of avidin or streptavidin and a biotinylated target-specific binding molecule;
(c) incubation of the complex formed in step (b) with cells.

12. Method according to claim 11, **characterized in that** the cells grow in cell culture in a suspension.

13. Method according to any one of claims 11 or 12, **characterized in that** the cells are blood cells or blood cell lines.

14. Method according to claim 13, **characterized in that** the cells are B- or T-cells or cell lines derived therefrom.

15. Reagent kit for introducing nucleic acid into cells, comprising at least
(a) dendrimers, wherein a proportion of the dendrimer molecules is biotinylated and a proportion of the dendrimer molecules is not biotinylated, wherein the proportion of dendrimer molecules that are biotinylated is 0.5 to 50% (mol/mol) of the total amount of dendrimer molecules; and
(b) avidin or streptavidin.

16. Reagent kit according to claim 15, **characterized in that** it additionally contains a biotinylated target-specific binding molecule and/or a nucleic acid.

17. Use of a reagent kit according to any one of claims 15 or 16 for introducing nucleic acid into cells.

18. Use according to claim 17, **characterized in that** the cells grow in cell culture in a suspension.

19. Method for producing a complex of nucleic acid and dendrimer, wherein a proportion of the dendrimer molecules is biotinylated and wherein the proportion of dendrimer molecules that is biotinylated is 0.5 to 50% (mol/mol) of the total quantity of dendrimer molecules, comprising the steps of
(a) reacting dendrimer with an equimolar amount of activated biotin or of an activated biotin derivative or a molar excess of activated biotin or activated biotin derivative under conditions in which covalent bonding takes place between dendrimer and biotin;
(b) mixing the biotinylated dendrimer formed in (a) with non-biotinylated dendrimer;
(c) incubation of the mixture formed in (b) with nucleic acid under conditions in which a complex of nucleic acid and dendrimer can be formed.

20. Method according to claim 19, wherein the molar excess of activated biotin or activated biotin derivate is a 2- to 64-fold molar excess.

21. Method according to any one of claims 19 to 20, wherein the molar excess of activated biotin or activated biotin derivative is a 2- to 12-fold molar excess.

22. Method according to any one of claims 19 to 21, wherein the mixing of the biotinylated dendrimer with non-biotinylated dendrimer is effected in a mass ratio of 1:1 to 1:256.

23. Method according to any one of claims 19 to 22, wherein the mixing of the biotinylated dendrimer with non-biotinylated dendrimer is effected in a mass ratio of 1:4 to 1:24.

24. Method for producing a dendrimer mixture, wherein a proportion of the dendrimer molecules is biotinylated and wherein the proportion of dendrimer molecules that is biotinylated is 0.5 to 50% (mol/mol) of the total quantity of dendrimer molecules, comprising the steps of
(a) reacting dendrimer with an equimolar amount of activated biotin or of an activated biotin derivative or a molar excess of activated biotin or activated biotin derivative under conditions in which covalent bonding takes place between dendrimer and biotin;
(b) mixing of the biotinylated dendrimer formed in (a) with non-biotinylated dendrimer.

25. Method according to claim 24, wherein the molar excess of activated biotin or activated biotin derivative is a 2- to 64-fold molar excess.

26. Method according to any one of claims 24 to 25, wherein the molar excess of activated biotin or activated biotin derivate is a 2- to 12-fold molar excess.

27. Method according to any one of claims 24 to 26, wherein the mixing of the biotinylated dendrimer with non-biotinylated dendrimer is effected in a mass ratio of 1:1 to 1:256.

28. Method according to any one of claims 24 to 27, wherein the mixing of the biotinylated dendrimer with non-biotinylated dendrimer is effected in a mass ratio of 1:4 to 1:24.

29. Dendrimer, wherein a proportion of the dendrimer molecules is biotinylated, obtainable by a method according to any one of claims 24 to 28.

30. Pharmaceutical composition comprising a composition according to any one of claims 1 to 10 or a dendrimer according to claim 29 and optionally a pharmaceutically acceptable carrier.

31. Use of a composition according to any one of claims 1 to 10 or of a dendrimer according to claim 29 for the preparation of a pharmaceutical composition for gene therapy.

## Revendications

1. Composition contenant un complexe d'acide nucléique et d'un dendrimère pour la transfection de cellules, dans laquelle une proportion des molécules dendrimères est biotinylée et une proportion des molécules dendrimères n'est pas biotinylée, **caractérisée en ce que** la proportion de molécules dendrimères qui est biotinylée est de 0,5 à 50 % (mol/mol) de la quantité totale de molécules dendrimères.

2. Composition selon la revendication 1, **caractérisée en ce que** la proportion de molécules dendrimères qui est biotinylée est de 5 à 20 % (mol/mol) de la quantité totale des molécules dendrimères.

3. Composition selon l'une quelconque des revendications précédentes, pouvant être obtenue par un procédé comprenant les étapes de :
(a) faire réagir un dendrimère avec une quantité équimolaire de biotine activée ou d'un dérivé de la biotine activé ou un excès molaire de biotine activée ou d'un dérivé de la biotine activé dans des conditions dans lesquelles une liaison covalente a lieu entre le dendrimère et la biotine ;
(b) mélanger le dendrimère biotinylé de l'étape (a) avec un dendrimère non biotinylé ;
(c) incuber le mélange formé dans l'étape (b) avec un acide nucléique dans des conditions dans lesquelles un complexe d'acide nucléique et de dendrimère peut se former.

4. Composition selon la revendication 3, dans laquelle l'excès molaire de la biotine activée ou d'un dérivé de la biotine activé est un excès molaire de 2 à 64 fois.

5. Composition selon l'une quelconque des revendications 3 à 4 dans laquelle l'excès molaire de la biotine activée ou d'un dérivé de la biotine activé est un excès molaire de 2 à 12 fois.

6. Composition selon l'une quelconque des revendications 3 à 5, dans laquelle le mélange du dendrimère biotinylé avec le dendrimère non biotinylé est réalisé selon un rapport pondéral de 1/1 à 1/256.

7. Composition selon l'une quelconque des revendications 3 à 6, dans laquelle le mélange du dendrimère biotinylé avec le dendrimère non biotinylé est réalisé selon un rapport pondéral de 1/4 à 1/24.

8. Composition selon l'une quelconque des revendications précédentes, **caractérisée en ce que** le complexe contient en outre de l'avidine ou de la streptavidine et une molécule de liaison spécifique à la cible biotinylée.

9. Composition selon l'une quelconque des revendications précédentes, **caractérisée en ce que** la molécule de liaison spécifique à la cible reconnaît une molécule de surface cellulaire.

10. Composition selon l'une quelconque des revendications précédentes, **caractérisée en ce que** la molécule de liaison spécifique à la cible est une transferrine ou un anticorps.

11. Procédé d'introduction d'un acide nucléique dans des cellules *in vitro* comprenant les étapes de :
(a) mélanger un acide nucléique avec une dendrimère, où une proportion des molécules dendrimères est biotinylée et où la proportion de molécules dendrimères qui est biotinylée constitue de 5 à 20 % (mol/mol) de la quantité totale de molécules dendrimères ;
(b) mélanger le complexe résultant d'acide nucléique et de dendrimère avec un deuxième complexe comprenant de l'avidine ou de la streptavidine et une molécule de liaison spécifique à la cible biotinylée ;
(c) incuber le complexe formé dans l'étape (b) avec les cellules.

12. Procédé selon la revendication 11, **caractérisé en ce que** les cellules se développent dans une culture cellulaire en suspension.

13. Procédé selon l'une quelconque des revendications 11 ou 12, **caractérisé en ce que** les cellules sont des cellules sanguines ou des lignées de cellules sanguines.

14. Procédé selon la revendication 13, **caractérisé en ce que** les cellules sont les cellules B ou T ou des lignées de cellules dérivées de celles-ci.

15. Kit de réactif pour l'introduction d'acide nucléique dans des cellules, comprenant au moins
(a) des dendrimères, dans lesquels une proportion des molécules dendrimères est biotinylée et une proportion des molécules dendrimères n'est pas biotinylée, où la proportion de molécules dendrimères qui est biotinylée est de 0,5 à 50 % (mol/mol) de la quantité totale des molécules dendrimères ; et
(b) de l'avidine ou de la streptavidine.

16. Kit de réactif selon la revendication 15, **caractérisé en ce qu'**il contient en outre une molécule de liaison spécifique à la cible biotinylée et/ou un acide nucléique.

17. Utilisation du kit de réactif selon l'une quelconque des revendications 15 à 16, pour l'introduction d'acide nucléique dans des cellules.

18. Utilisation selon la revendication 17, **caractérisée en ce que** les cellules se développent dans une culture cellulaire en suspension.

19. Procédé de production d'un complexe d'acide nucléique et de dendrimère, dans lequel une proportion des molécules dendrimères est biotinylée et où la proportion de molécules dendrimères qui est biotinylée constitue de 0,5 à 50 % (mol/mol) de la quantité totale de molécules dendrimères, comprenant les étapes de :
(a) faire réagir un dendrimère avec une quantité équimolaire de biotine activée ou d'un dérivé de la biotine activé ou un excès molaire de biotine activée ou d'un dérivé de la biotine activé dans des conditions dans lesquelles une liaison covalente a lieu entre le dendrimère et la biotine ;
(b) mélanger le dendrimère biotinylé de l'étape (a) avec un dendrimère non biotinylé ;
(c) incuber le mélange formé dans l'étape (b) avec un acide nucléique dans des conditions dans lesquelles un complexe d'acide nucléique et de dendrimère peut se former.

20. Procédé selon la revendication 19, dans lequel l'excès molaire de la biotine activée ou d'un dérivé de la biotine activé est un excès molaire de 2 à 64 fois.

21. Procédé selon l'une quelconque des revendications 19 à 20, dans lequel l'excès molaire de la biotine activée ou d'un dérivé de la biotine activé est un excès molaire de 2 à 12 fois.

22. Procédé selon l'une quelconque des revendications 19 à 21, dans lequel le mélange du dendrimère biotinylé avec le dendrimère non biotinylé est réalisé selon un rapport pondéral de 1/1 à 1/256.

23. Procédé selon l'une quelconque des revendications 19 à 22, dans lequel le mélange du dendrimère biotinylé avec le dendrimère non biotinylé est réalisé selon un rapport pondéral de 1/4 à 1/24.

24. Procédé de production d'un mélange de dendrimères, dans lequel une proportion des molécules dendrimères est biotinylée et où la proportion de molécules dendrimères qui est biotinylée constitue de 0,5 à 50 % (mol/mol) de la quantité totale des molécules dendrimères, comprenant les étapes de :
(a) faire réagire un dendrimère avec une quantité équimolaire de biotine activée ou d'un dérivé de la biotine activé ou un excès molaire de biotine activée ou d'un dérivé de la biotine activé dans des conditions dans lesquelles une liaison covalente a lieu entre le dendrimère et la biotine ; et
(b) mélanger le dendrimère biotinylé de l'étape (a) avec un dendrimère non biotinylé.

25. Procédé selon la revendication 24, dans lequel l'excès molaire de la biotine activée ou d'un dérivé de la biotine activé est un excès molaire de 2 à 64 fois.

26. Procédé selon l'une quelconque des revendications 24 à 25, dans lequel l'excès molaire de la biotine activée ou d'un dérivé de la biotine activé est un excès molaire de 2 à 12 fois.

27. Procédé selon l'une quelconque des revendications 24 à 26, dans lequel le mélange du dendrimère biotinylé avec le dendrimère non biotinylé est réalisé selon un rapport pondéral de 1/1 à 1/256.

28. Procédé selon l'une quelconque des revendications 24 à 27, dans lequel le mélange du dendrimère biotinylé avec le dendrimère non biotinylé est réalisé selon un rapport pondéral de 1/4 à 1/24.

29. Dendrimère, dans lequel une proportion des molécules dendrimères est biotinylée, pouvant être obtenu par un procédé selon l'une quelconque des revendications 24 à 28.

30. Composition pharmaceutique comprenant une composition selon l'une quelconque des revendications 1 à 10 ou un dendrimère selon la revendication 29 et éventuellement un support pharmaceutiquement acceptable.

31. Utilisation d'une composition selon l'une quelconque des revendications 1 à 10 ou d'un dendrimère selon la revendication 29 pour la préparation d'une composition pharmaceutique pour une thérapie génique.
